(19) Europäisches Patentamt
European Patent Office
Office européen des brevets

(11) EP 3 855 445 A1

(12) # EUROPEAN PATENT APPLICATION

(43) Date of publication:
**28.07.2021 Bulletin 2021/30**

(21) Application number: **20205387.2**

(22) Date of filing: **03.11.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.01.2020 JP 2020009953**

(51) Int Cl.:
***G16C 20/40*** *(2019.01)*

(71) Applicant: **FUJITSU LIMITED**
**Kanagawa 211-8588 (JP)**

(72) Inventor: **JIPPO, Hideyuki**
**Kawasaki-shi, Kanagawa 211-8588 (JP)**

(74) Representative: **Haseltine Lake Kempner LLP**
**Cheapside House**
**138 Cheapside**
**London**
**EC2V 6BJ (GB)**

(54) **SIMILARITY CALCULATION DEVICE, SIMILARITY CALCULATION METHOD, AND PROGRAM**

(57) A similarity calculation device calculates a similarity between a first material and a second material and includes: a creation unit that creates a conflict graph that is a graph that has a plurality of nodes made up of combinations of respective atoms that constitute the first material and respective atoms that constitute the second material, and an edge formed between two nodes among the plurality of nodes, and that has an edge between two nodes when the nodes are compared and are not identical to each other, and has no edge between two nodes when the nodes are compared and are identical to each other; a search unit that searches for a maximum independent set in the conflict graph by executing a ground state search using an annealing method; and a computation unit that computes the similarity between the first material and the second material based on the maximum independent set. The plurality of nodes of the conflict graph is each made up of a combination of two atoms that have an atom type that is same between the first material and the second material and the atom type is subdivided more finely than elemental species.

FIG. 15

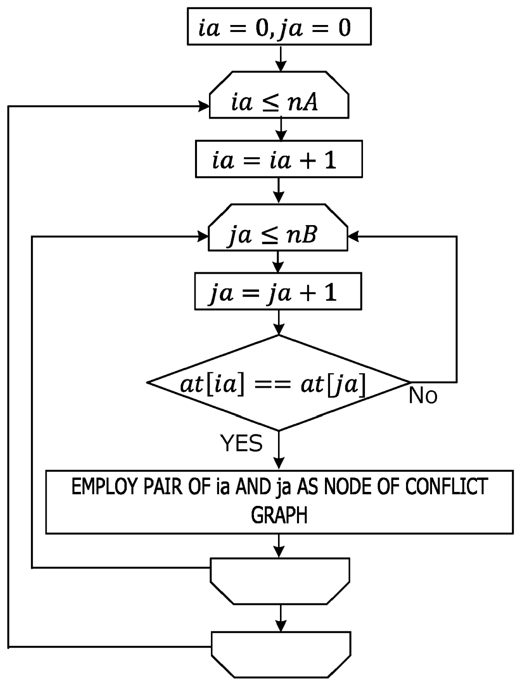


EP 3 855 445 A1

## Description

FIELD

The embodiments discussed herein are related to a similarity calculation device, a similarity calculation method, and a program.

BACKGROUND

Generally, compounds (molecules) having similar structures are expected to have similar characteristics (properties). This similar property principle that "similar compounds have similar properties" is widely used, for example, when a compound having a predetermined property is designed by predicting the properties of compounds, or when a compound having a predetermined property is searched for by screening a database of compounds.

When the similar property principle is used, for example, it can be predicted that, by utilizing an existing compound as a query compound, a compound with similarity (a compound having a structure similar to the structure of the query compound) retrieved from a database has the same function (characteristics and physical properties) as the query compound. Furthermore, when a new compound is utilized as a query compound, the characteristic value of a new chemical substance can also be predicted by searching a database for a compound having a structure similar to the structure of the query compound.

Here, the search for compounds having similar structures to each other can be performed by, for example, evaluating the similarity in structure between the compounds and specifying a compound having a high similarity in structure as a similar compound.

Although a variety of techniques have been proposed as techniques for evaluating the similarity in structure between compounds, for example, the fingerprint method is widely used. In the fingerprint method, for example, whether or not the substructure of the query compound is contained in the compound to be compared is represented by 0 or 1, and the similarity is evaluated.

Furthermore, as a technique of evaluating the similarity in structure, a technique of searching for a substructure common to compounds by solving the maximum independent set problem in the conflict graph represented by an Ising model equation with an annealing machine or the like is also proposed (for example, refer to Non-Patent Document 1).

However, this proposed technology has room for examination in terms of the accuracy of structural similarity to be computed. In addition, in this proposed technology, the number of bits to be used for the annealing machine is raised as the number of atoms constituting the compound increases.

Hernandez, Maritza; Zaribafiyan, Arman; Aramon, Maliheh; Naghibi, Mohammad, "A Novel Graph-based Approach for Determining Molecular Similarity", arXiv:1601.06693 (https://arxiv.org/pdf/1601.06693.pdf) (Non-Patent Document 1) is disclosed as related art.

In one aspect, an object of the present application is to provide a similarity calculation device, a similarity calculation method, and a program that are excellent in the accuracy of structural similarity to be computed and capable of reducing the number of bits to be used for the calculation.

SUMMARY

According to one aspect of the embodiments, a similarity calculation device calculates a similarity between a first material and a second material and includes: a creation unit that creates a conflict graph that is a graph that has a plurality of nodes made up of combinations of respective atoms that constitute the first material and respective atoms that constitute the second material, and an edge formed between two nodes among the plurality of nodes, and that has an edge between two nodes when the nodes are compared and are not identical to each other, and has no edge between two nodes when the nodes are compared and are identical to each other; a search unit that searches for a maximum independent set in the conflict graph by executing a ground state search using an annealing method; and a computation unit that computes the similarity between the first material and the second material based on the maximum independent set. The plurality of nodes of the conflict graph is each made up of a combination of two atoms that have an atom type that is same between the first material and the second material and the atom type is subdivided more finely than elemental species.

According to another aspect of the embodiments, a similarity calculation method calculates a similarity between a first material and a second material and includes: creating a conflict graph that is a graph that has a plurality of nodes made up of combinations of respective atoms that constitute the first material and respective atoms that constitute the second material, and an edge formed between two nodes among the plurality of nodes, and that has an edge between two nodes when the nodes are compared and are not identical to each other, and has no edge between two nodes when the nodes are compared and are identical to each other; searching for a maximum independent set in the conflict graph

by executing a ground state search using an annealing method; and computing the similarity between the first material and the second material based on the maximum independent set. The plurality of nodes of the conflict graph is each made up of a combination of two atoms that have an atom type that is same between the first material and the second material and the atom type is subdivided more finely than elemental species.

According to still another aspect of the embodiments, a program causing a computer to perform a creation process of: creating a conflict graph that is a graph that has a plurality of nodes made up of combinations of respective atoms that constitute the first material and respective atoms that constitute the second material, and an edge formed between two nodes among the plurality of nodes, and that has an edge between two nodes when the nodes are compared and are not identical to each other, and has no edge between two nodes when the nodes are compared and are identical to each other; searching for a maximum independent set in the conflict graph by executing a ground state search using an annealing method; and computing the similarity between the first material and the second material based on the maximum independent set. The plurality of nodes of the conflict graph is each made up of a combination of two atoms that have an atom type that is same between the first material and the second material and the atom type is subdivided more finely than elemental species.

In one aspect, the present application may provide a similarity calculation device, a similarity calculation method, and a program that are excellent in the accuracy of structural similarity to be computed and capable of reducing the number of bits to be used for the calculation.

BRIEF DESCRIPTION OF DRAWINGS

FIG. 1 is a diagram of prior art illustrating an example of how acetic acid and methyl acetate are expressed as graphs;
FIG. 2 is a diagram of the prior art illustrating exemplary combinations in a case where the same elements in a molecule A and a molecule B are combined and employed as nodes of a conflict graph;
FIG. 3 is a diagram of the prior art illustrating an exemplary rule for creating an edge in the conflict graph;
FIG. 4 is a diagram of the prior art illustrating an exemplary conflict graph of the molecule A and the molecule B;
FIG. 5 is a diagram of the prior art illustrating an exemplary maximum independent set in a graph;
FIG. 6 is a diagram of the prior art illustrating an exemplary flow in a case where a maximum common substructure of the molecule A and the molecule B is worked out (a maximum independent set problem is solved) by working out a maximum independent set in a conflict graph;
FIG. 7 is an explanatory diagram for explaining an exemplary prior technique of searching for a maximum independent set in a graph of which the number of nodes is six;
FIG. 8 is an explanatory diagram for explaining an exemplary prior technique of searching for a maximum independent set in a graph of which the number of nodes is six;
FIG. 9 is a diagram of the prior art illustrating an exemplary maximum independent set in a conflict graph;
FIG. 10 is a diagram representing an example of expressing acetic acid and methyl acetate as graphs, based on the atom type of general AMBER force field (GAFF);
FIG. 11 is a diagram representing an example of creating nodes of a conflict graph from graphs of acetic acid and methyl acetate based on the GAFF atom type;
FIG. 12 is a conflict graph created from the nodes illustrated in FIG. 11;
FIG. 13 is a diagram for explaining an exemplary sequence from reading the molecular structure to searching for a maximum independent set, using acetic acid and methyl acetate as examples (part 1);
FIG. 14 is a diagram for explaining an exemplary sequence from reading the molecular structure to searching for a maximum independent set, using acetic acid and methyl acetate as examples (part 2);
FIG. 15 is a diagram for explaining an exemplary sequence from reading the molecular structure to searching for a maximum independent set, using acetic acid and methyl acetate as examples (part 3);
FIG. 16 is a diagram for explaining an exemplary sequence from reading the molecular structure to searching for a maximum independent set, using acetic acid and methyl acetate as examples (part 4);
FIG. 17 is a diagram for explaining an exemplary sequence from reading the molecular structure to searching for a maximum independent set, using acetic acid and methyl acetate as examples (part 5);
FIG. 18 is a diagram for explaining an exemplary sequence from reading the molecular structure to searching for a maximum independent set, using acetic acid and methyl acetate as examples (part 6);
FIG. 19 is a diagram for explaining an exemplary sequence from reading the molecular structure to searching for a maximum independent set, using acetic acid and methyl acetate as examples (part 7);
FIG. 20 is a diagram representing an exemplary configuration of a similarity calculation device disclosed in the present application;
FIG. 21 is a diagram representing another exemplary configuration of the similarity calculation device disclosed in

the present application;
FIG. 22 is a diagram representing another exemplary configuration of the similarity calculation device disclosed in the present application;
FIG. 23 is a diagram representing another exemplary configuration of the similarity calculation device disclosed in the present application;
FIG. 24 is a diagram illustrating an exemplary functional configuration as an embodiment of the similarity calculation device disclosed in the present application;
FIG. 25 is a flowchart of an embodiment of similarity calculation disclosed in the present application;
FIG. 26 is a diagram illustrating an exemplary functional configuration of an optimizing device (control unit) used in an annealing method;
FIG. 27 is a block diagram illustrating an example of a transition control unit at a circuit level;
FIG. 28 is a diagram illustrating an exemplary operation flow of the transition control unit;
FIG. 29 is a diagram illustrating a chemical structure of linalool;
FIG. 30 is a diagram representing the number of bits in a conventional example; and
FIG. 31 is a diagram representing the number of bits in an example.

DESCRIPTION OF EMBODIMENTS

(Similarity Calculation Device, Similarity Calculation Method, Program)

A similarity calculation device disclosed in the present application is a device that calculates the similarity between a first material and a second material.
The similarity calculation device includes a creation unit, a search unit, and a computation unit, and further includes other units depending on the situation.
The creation unit creates a conflict graph.
The conflict graph is a graph that has a plurality of nodes made up of combinations of respective atoms that constitute the first material and respective atoms that constitute the second material, and an edge formed between two nodes among the plurality of nodes, and that has an edge between two nodes when the nodes are compared and are not identical to each other, and has no edge between two nodes when the nodes are compared and are identical to each other.
The search unit searches for a maximum independent set in the conflict graph by executing a ground state search using the annealing method.
The computation unit computes the similarity between the first material and the second material based on the maximum independent set.
Here, the plurality of nodes of the conflict graph is each made up of a combination of two atoms that have the same atom type, which is subdivided more finely than the elemental species, between the first material and the second material.
A similarity calculation method disclosed in the present application is a method of calculating the similarity between the first material and the second material.
The similarity calculation method includes a creation process, a search process, and a computation process, and further includes other processes depending on the situation.
The creation process is a process of creating a conflict graph.
The conflict graph is a graph that has a plurality of nodes made up of combinations of respective atoms that constitute the first material and respective atoms that constitute the second material, and an edge formed between two nodes among the plurality of nodes, and that has an edge between two nodes when the nodes are compared and are not identical to each other, and has no edge between two nodes when the nodes are compared and are identical to each other.
The search process is a process of searching for a maximum independent set in the conflict graph by executing a ground state search using the annealing method.
The computation process is a process of computing the similarity between the first material and the second material based on the maximum independent set.
Here, the plurality of nodes of the conflict graph is each made up of a combination of two atoms that have the same atom type, which is subdivided more finely than the elemental species, between the first material and the second material.
A program disclosed in the present application includes causing a computer to perform the creation process.
The creation process is a process of creating a conflict graph.
The conflict graph is a graph that has a plurality of nodes made up of combinations of respective atoms that constitute the first material and respective atoms that constitute the second material, and an edge formed between two

nodes among the plurality of nodes, and that has an edge between two nodes when the nodes are compared and are not identical to each other, and has no edge between two nodes when the nodes are compared and are identical to each other.

Here, the plurality of nodes of the conflict graph is each made up of a combination of two atoms that have the same atom type, which is subdivided more finely than the elemental species, between the first material and the second material.

First, prior to describing the details of the technology disclosed in the present application, description will be given of a prior technique of searching for a substructure common to materials to be compared and computing the similarity between the materials by solving a maximum independent set problem in a conflict graph.

When the similarity in structure between compounds is computed by solving the maximum independent set problem in the conflict graph, the compounds are treated by being expressed as graphs. Here, to express a compound as a graph means to represent the structure of the compound using, for example, information on the types of atoms (element) in the compound and information on the bonding state between the respective atoms.

The structure of a compound can be represented using, for example, expression in a MOL format or a structure data file (SDF) format. Usually, the SDF format means a single file obtained by collecting structural information on a plurality of compounds expressed in the MOL format. Furthermore, besides the MOL format structural information, the SDF format file is capable of treating additional information (for example, the catalog number, the Chemical Abstracts Service (CAS) number, the molecular weight, or the like) for each compound. Such a structure of the compound can be expressed as a graph in a comma-separated value (CSV) format in which, for example, "atom 1 (name), atom 2 (name), element information on atom 1, element information on atom 2, bond order between atom 1 and atom 2" are contained in a single row.

In the following, a method of creating the conflict graph will be described by taking a case of creating a conflict graph of acetic acid ($CH_3COOH$) and methyl acetate ($CH_3COOCH_3$) as an example.

First, acetic acid (hereinafter sometimes referred to as "molecule A") and methyl acetate (hereinafter sometimes referred to as "molecule B") are expressed as graphs, and are given as illustrated in FIG. 1. In FIG. 1, atoms that form acetic acid are indicated by A1, A2, A3, and A5, and atoms that form methyl acetate are indicated by B1 to B5. Furthermore, in FIG. 1, A1, A2, B1, B2, and B4 indicate carbon, and A3, A5, B3, and B5 indicate oxygen, while a single bond is indicated by a thin solid line and a double bond is indicated by a thick solid line. Note that, in the example illustrated in FIG. 1, atoms other than hydrogen are selected and expressed as graphs, but when a compound is expressed as a graph, all atoms including hydrogen may be selected and expressed as a graph.

Next, the vertices (atoms) of the molecules A and B expressed as graphs are combined to create vertices (nodes) of the conflict graph. At this time, as illustrated in FIG. 2, the same elements in the molecules A and B are combined and employed as nodes of the conflict graph. In the example illustrated in FIG. 2, combinations of A1, A2, B1, B2, and B4 that represent carbon and combinations of A3, A5, B3, and B5 that represent oxygen are employed as nodes of the conflict graph.

In the example in FIG. 2, six nodes are created by combinations of carbons of the molecule A and carbons of the molecule B, and four nodes are created by combinations of oxygens of the molecule A and oxygens of the molecule B; accordingly, the number of nodes in the conflict graph created from the molecules A and B expressed as graphs is given as ten.

Subsequently, edges (branches or sides) in the conflict graph are created. At this time, two nodes are compared, and when the nodes are constituted by atoms in different situations from each other (for example, the atomic number, the presence or absence of bond, the bond order, or the like), an edge is created between these two nodes. On the other hand, when two nodes are compared and the nodes are constituted by atoms in the same situation, no edge is created between these two nodes.

Here, a rule for creating the edge in the conflict graph will be described with reference to FIG. 3.

First, in the example illustrated in FIG. 3, whether or not an edge is created between the node [A1B1] and the node [A2B2] will be described. As can be seen from the structure of the molecule A expressed as a graph in FIG. 3, the carbon A1 of the molecule A included in the node [A1B1] and the carbon A2 of the molecule A included in the node [A2B2] are bonded (single bonded) to each other. Likewise, the carbon B1 of the molecule B included in the node [A1B1] and the carbon B2 of the molecule B included in the node [A2B2] are bonded (single bonded) to each other. For example, the situation of bonding between the carbons A1 and A2 and the situation of bonding between the carbons B1 and B2 are identical to each other.

In this manner, in the example in FIG. 3, the situation of the carbons A1 and A2 in the molecule A and the situation of the carbons B1 and B2 in the molecule B are identical to each other, and the nodes [A1B1] and [A2B2] are deemed as nodes constituted by atoms in identical situations to each other. Therefore, in the example illustrated in FIG. 3, no edge is created between the nodes [A1B1] and [A2B2].

Next, in the example illustrated in FIG. 3, whether or not an edge is created between the node [A1B4] and the node [A2B2] will be described. As can be seen from the structure of the molecule A expressed as a graph in FIG. 3, the

carbon A1 of the molecule A included in the node [A1B4] and the carbon A2 of the molecule A included in the node [A2B2] are bonded (single bonded) to each other. On the other hand, as can be seen from the structure of the molecule B expressed as a graph, the carbon B4 of the molecule B included in the node [A1B4] and the carbon B2 of the molecule B included in the node [A2B2] have the oxygen B3 sandwiched between the carbons B4 and B2, and are not directly bonded. For example, the situation of bonding between the carbons A1 and A2 and the situation of bonding between the carbons B4 and B2 are different from each other.

Thus, in the example in FIG. 3, the situation of the carbons A1 and A2 in the molecule A and the situation of the carbons B4 and B2 in the molecule B are different from each other, and the nodes [A1B4] and [A2B2] are deemed as nodes constituted by atoms in different situations from each other. Therefore, in the example illustrated in FIG. 3, an edge is created between the nodes [A1B4] and [A2B2].

In this manner, the conflict graph can be created based on the rule that, when nodes are constituted by atoms in different situations, an edge is created between these nodes, and when nodes are constituted by atoms in the same situation, no edge is created between these nodes.

FIG. 4 is a diagram illustrating an exemplary conflict graph of the molecules A and B. As illustrated in FIG. 4, for example, in the nodes [A2B2] and [A5B5], the situation of bonding between the carbon A2 and the oxygen A5 in the molecule A and the situation of bonding between the carbons B2 and B5 in the molecule B are identical to each other. Therefore, the nodes [A2B2] and [A5B5] are deemed as nodes constituted by atoms in identical situations to each other, and thus no edge has been created between the nodes [A2B2] and [A5B5].

Here, the edge of the conflict graph can be created, for example, based on chemical structure data of two compounds for which the similarity in structure is to be computed. For example, when chemical structure data of compounds is input using an SDF format file, edges of the conflict graph can be created (specified) by performing calculations using a calculator such as a computer based on information contained in the SDF format file.

Next, a method of solving the maximum independent set problem in the created conflict graph in exemplary prior art as described in Non-Patent Document 1 will be described.

A maximum independent set (MIS) in the conflict graph means a set that includes the largest number of nodes that have no edges between the nodes among sets of nodes that constitute the conflict graph. For example, the maximum independent set in the conflict graph means a set that has the maximum size (number of nodes) among sets formed by nodes that have no edges between the nodes with each other.

FIG. 5 is a diagram illustrating an exemplary maximum independent set in a graph. In FIG. 5, nodes included in a set are marked with a reference sign of "1", and nodes not included in any set are marked with a reference sign of "0"; for instances where edges are present between nodes, the nodes are connected by solid lines, and for instances where no edges are present, the nodes are connected by dotted lines. Note that, here, as illustrated in FIG. 5, a graph of which the number of nodes is six will be described as an example for simplification of explanation.

In the example illustrated in FIG. 5, among sets constituted by nodes that have no edges between the nodes, there are three sets having the maximum number of nodes, and the number of nodes in each of these sets is three. For example, in the example illustrated in FIG. 5, three sets surrounded by the one-dot chain line are given as the maximum independent sets in the graph.

Here, as described above, the conflict graph is created based on the rule that, when nodes are constituted by atoms in different situations, an edge is created between these nodes, and when nodes are constituted by atoms in the same situation, no edge is created between these nodes. Therefore, in the conflict graph, working out the maximum independent set, which is a set having the maximum number of nodes among sets constituted by nodes that have no edges between the nodes, is synonymous with working out the largest substructure among substructures common to two molecules. For example, the largest common substructure of two molecules can be specified by working out the maximum independent set in the conflict graph.

Thus, by expressing two molecules as graphs, creating a conflict graph based on the structures of the molecules expressed as graphs, and working out the maximum independent set in the conflict graph, the maximum common substructure of the two molecules can be worked out.

FIG. 6 illustrates an exemplary flow in a case where a maximum common substructure of the molecule A (acetic acid) and the molecule B (methyl acetate) is worked out (a maximum independent set problem is solved) by working out the maximum independent set in the conflict graph. As illustrated in FIG. 6, a conflict graph is created in such a manner that the molecules A and B are each expressed as a graph, the same elements are combined and employed as a node, and an edge is formed according to the situation of atoms constituting the node. Then, by working out the maximum independent set in the created conflict graph, the maximum common substructure of the molecules A and B can be worked out.

Here, an exemplary specific method for working out (searching for) the maximum independent set in the conflict graph will be described.

The search for the maximum independent set in the conflict graph can be performed, for example, by using a Hamiltonian in which minimizing means searching for the maximum independent set. For example, the search can be

performed by using a Hamiltonian (H) indicated by following Formula (1).
[Mathematical Formula 1]

$$H = -\alpha \sum_{i=0}^{n-1} b_i x_i + \beta \sum_{i,j=0}^{n-1} w_{ij} x_i x_j \quad \text{... Formula (1)}$$

Here, in above Formula (1), n denotes the number of nodes in the conflict graph, and bi denotes a numerical value that represents a bias for an i-th node.

Moreover, $w_{ij}$ has a positive non-zero number when there is an edge between the i-th node and a j-th node, and has zero when there is no edge between the i-th node and the j-th node.

Furthermore, $x_i$ denotes a binary variable that represents that the i-th node has 0 or 1, and $x_j$ denotes a binary variable that represents that the j-th node has 0 or 1.

Note that $\alpha$ and $\beta$ denote positive numbers.

The relationship between the Hamiltonian represented by above Formula (1) and the search for the maximum independent set will be described in more detail. Above Formula (1) is a Hamiltonian that represents an Ising model equation in the quadratic unconstrained binary optimization (QUBO) format.

In above Formula (1), when $x_i$ has 1, it means that the i-th node is included in a set that is a candidate for the maximum independent set, and when $x_i$ has 0, it means that the i-th node is not included in a set that is a candidate for the maximum independent set. Likewise, in above Formula (1), when $x_j$ has 1, it means that the j-th node is included in a set that is a candidate for the maximum independent set, and when $x_j$ has 0, it means that the j-th node is not included in a set that is a candidate for the maximum independent set.

Therefore, in above Formula (1), by searching for a combination in which as many nodes as possible have the state of 1 under the constraint that there is no edge between nodes whose states are designated as 1 (bits are designated as 1), the maximum independent set can be retrieved.

Here, each term in above Formula (1) will be described.

The first term on the right side of above Formula (1) (the term with the coefficient of $-\alpha$) is a term whose value becomes smaller as the number of i whose $x_i$ has 1 rises (the number of nodes included in a set that is a candidate for the maximum independent set rises). Note that the value of the first term on the right side of above Formula (1) becoming smaller means that a larger negative number is given. Thus, in above Formula (1), the value of the Hamiltonian (H) becomes smaller when much nodes have the bit of 1, due to the action of the first term on the right side.

The second term on the right side of above Formula (1) (the term with the coefficient of $\beta$) is a term of the penalty whose value becomes larger when there is an edge between nodes whose bits have 1 (when $w_{ij}$ has a positive non-zero number). For example, the second term on the right side of above Formula (1) has 0 when there is no instance where an edge is present between nodes whose bits have 1, and has a positive number in other cases. Thus, in above Formula (1), the value of the Hamiltonian (H) becomes larger when there is an edge between nodes whose bits have 1, due to the action of the second term on the right side.

As described above, above Formula (1) has a smaller value when much nodes have the bit of 1, and has a larger value when there is an edge between the nodes whose bits have 1; accordingly, it can be said that minimizing above Formula (1) means searching for the maximum independent set.

Here, the relationship between the Hamiltonian represented by above Formula (1) and the search for the maximum independent set will be described using an example with reference to the drawings.

A case where the bit is set in each node as in the example illustrated in FIG. 7 in a graph of which the number nodes is six will be considered. In the example in FIG. 7, as in FIG. 5, for instances where edges are present between nodes, the nodes are connected by solid lines, and for instances where no edges are present, the nodes are connected by dotted lines.

For the example in FIG. 7, assuming in above Formula (1) that bi has 1, and $w_{ij}$ has 1 when there is an edge between the i-th node and the j-th node, above Formula (1) is as follows.

[Mathematical Formula 2]

$$\begin{aligned} H = & -\alpha(x_0 + x_1 + x_2 + x_3 + x_4 + x_5) \\ & + \beta(\lambda_{01}x_0x_1 + \lambda_{02}x_0x_2 + \lambda_{03}x_0x_3 + \lambda_{04}x_0x_4 + \lambda_{05}x_0x_5 + \cdots) \\ = & -\alpha(1 + 0 + 1 + 0 + 1 + 0) \\ & + \beta(1 * 1 * 0 + 0 * 1 * 1 + 0 * 1 * 0 + 0 * 1 * 1 + 0 * 1 * 0 + \cdots) \\ = & -3\alpha \end{aligned}$$

In this manner, in the example in FIG. 7, when there is no instance where an edge is present between nodes whose bits have 1 (when there is no contradiction as an independent set), the second term on the right side has 0, and the value of the first term is given as the value of the Hamiltonian as it is.

Next, a case where the bit is set in each node as in the example illustrated in FIG. 8 will be considered. As in the example in FIG. 7, assuming in above Formula (1) that bi has 1, and $w_{ij}$ has 1 when there is an edge between the i-th node and the j-th node, above Formula (1) is as follows.

[Mathematical Formula 3]

$$\begin{aligned} H = & -\alpha(x_0 + x_1 + x_2 + x_3 + x_4 + x_5) \\ & + \beta(\lambda_{01}x_0x_1 + \lambda_{02}x_0x_2 + \lambda_{03}x_0x_3 + \lambda_{04}x_0x_4 + \lambda_{05}x_0x_5 + \cdots) \\ = & -\alpha\left(1 + \underline{1} + 1 + 0 + 1 + 0\right) \\ & + \beta\left(1 * 1 * \underline{1} + 0 * 1 * 1 + 0 * 1 * 0 + 0 * 1 * 1 + 0 * 1 * 0 + \cdots\right) \\ = & -4\alpha + 5\beta \end{aligned}$$

In this manner, in the example in FIG. 8, since there is an instance where an edge is present between nodes whose bits have 1, the second term on the right side does not have 0, and the value of the Hamiltonian is given as the sum of the two terms on the right side. Here, in the examples illustrated in FIGs. 7 and 8, for example, when $\alpha > 5\beta$ is assumed, $-3\alpha < -4\alpha + 5\beta$ is satisfied, and accordingly, the value of the Hamiltonian in the example in FIG. 7 is smaller than the value of the Hamiltonian in the example in FIG. 8. In the example in FIG. 7, a set of nodes that has no contradiction as the maximum independent set is obtained, and it can be seen that the maximum independent set can be retrieved by searching for a combination of nodes in which the value of the Hamiltonian in above Formula (1) becomes smaller.

Next, a method of computing the similarity in structure between molecules based on the retrieved maximum independent set in exemplary prior art as described in Non-Patent Document 1 will be described.

The similarity in structure between molecules can be computed, for example, using following Formula (2).
[Mathematical Formula 4]

$$S(G_A, G_B) = \delta \max\left\{\frac{|V_C^A|}{|V_A|}, \frac{|V_C^B|}{|V_B|}\right\} + (1-\delta)\min\left\{\frac{|V_C^A|}{|V_A|}, \frac{|V_C^B|}{|V_B|}\right\} \quad \text{... Formula (2)}$$

Here, in above Formula (2), $S(G_A, G_B)$ represents the similarity between a first molecule expressed as a graph (for example, the molecule A) and a second molecule expressed as a graph (for example, the molecule B), is represented as 0 to 1, and means that the closer to 1, the higher the similarity.

Furthermore, $V_A$ represents the total number of node atoms of the first molecule expressed as a graph, and $V_c^A$ represents the number of node atoms included in the maximum independent set of the conflict graph among the node atoms of the first molecule expressed as a graph. Note that the node atom means an atom at the vertex of the molecule expressed as a graph.

Moreover, $V_B$ represents the total number of node atoms of the second molecule expressed as a graph, and $V_c^B$ represents the number of node atoms included in the maximum independent set of the conflict graph among the node atoms of the second molecule expressed as a graph.

The sign $\delta$ denotes a number from 0 to 1.

In addition, in above Formula (2), max{A, B} means to select a larger value from among A and B, and min{A, B} means to select a smaller value from among A and B.

Here, as in FIG. 1 and other drawings, a method of computing the similarity will be described taking acetic acid

(molecule A) and methyl acetate (molecule B) as examples.

In the conflict graph illustrated in FIG. 9, the maximum independent set is constituted by four nodes: a node [A1B1], a node [A2B2], a node [A3B3], and a node [A5B5]. Thus, in the example in FIG. 9, $|V_A|$ is given as 4, $|V_c^A|$ is given as 4, $|V_B|$ is given as 5, and $|V_c^B|$ is given as 4. Furthermore, in this example, when it is assumed that δ has 0.5 and the average of the first molecule and the second molecule is taken (treated equally), above Formula (2) is as follows.

[Mathematical Formula 5]

$$S(G_A, G_B) = 0.5 * \max\left\{\frac{4}{4}, \frac{4}{5}\right\} + (1 - 0.5) * \min\left\{\frac{4}{4}, \frac{4}{5}\right\}$$
$$= 0.5 * \frac{4}{4} + (1 - 0.5) * \frac{4}{5} = 0.9$$

In this manner, in the example in FIG. 9, the similarity in structure between the molecules is computed as 0.9 based on above Formula (2).

As described above, in exemplary prior art as described in Non-Patent Document 1, the similarity in structure between compounds (molecules) is computed using above Formulas (1) and (2).

However, in such prior art, as illustrated in FIG. 2, the same elements in the molecules A and B are combined and employed as nodes of the conflict graph. Therefore, when the nodes of the conflict graph are created, the states of the atoms other than the elements are not taken into account, and there is room for improvement in the accuracy of similarity; besides, if the number of atoms that constitute the compound increases, the number of bits to be used for the calculation is raised.

In view of this, the present inventors have found that, by searching the conflict graph for the maximum independent set, and when calculating the similarity, configuring a node of the conflict graph from a combination of two atoms that have the same atom type, which is subdivided more finely than the elemental species, between a first material and a second material, the accuracy of similarity may be improved, and the number of nodes may be reduced (which means that the number of bits to be used for the calculation may be reduced).

When a node of the conflict graph is configured from a combination of two atoms that have the same atom type, which is subdivided more finely than the elemental species, between the first material and the second material, the atom type includes, for example, the orbital hybridization, the type of aromaticity, the type of chemical environment of the atom, and the like. An example of this will be described.

Furthermore, for example, a plurality of nodes of the conflict graph is each made up of a combination of two atoms that are the same in the atom type and bond type between the first material and the second material. The bond type includes, for example, whether or not the concerned combination is included in an aromatic ring and whether or not the concerned combination has a covalent, ionic or coordinate bond.

FIG. 10 is a diagram illustrating an example of how acetic acid and methyl acetate are expressed as graphs.

In FIG. 10, atoms that form acetic acid are indicated by A1, A2, A3, and A5, and atoms that form methyl acetate are indicated by B1 to B5. Furthermore, in FIG. 10, A1, A2, B1, B2, and B4 indicate carbon, and A3, A5, B3, and B5 indicate oxygen, while a single bond is indicated by a thin solid line and a double bond is indicated by a thick solid line. Note that, in the example illustrated in FIG. 10, atoms other than hydrogen are selected and expressed as graphs, but when a compound is expressed as a graph, all atoms including hydrogen may be selected and expressed as a graph. This graph is the same as the graph illustrated in FIG. 1 up to this point. However, in FIG. 10, carbon and oxygen are further subdivided based on the orbital hybridization, the aromaticity, and the chemical environment. In FIG. 10, the atom type is subdivided based on the atom type of general AMBER force field (GAFF). The GAFF atom type is introduced, for example, in Table 1 or the like of the following document.

Document: WANG, JUNMEI; WOLF, ROMAIN M.; CALDWELL, JAMES W.; KOLLMAN, PETER A.; CASE, DAVID A., "Development and Testing of a General Amber Force Field", Journal of Computational Chemistry, Vol. 25, No. 9

Here, in FIG. 10, "c3" represents $sp^3$ carbon, "c2" represents aliphatic $sp^2$ carbon, "o" represents $sp^2$ oxygen in C=O or $COO^-$, "oh" represents $sp^3$ oxygen in the hydroxyl group, and "os" represents $sp^3$ oxygen in ether or ester.

The graph of acetic acid and the graph of methyl acetate in FIG. 10 have these pieces of information on the atom type.

Next, the vertices (atoms) of the molecules A and B expressed as graphs are combined to create vertices (nodes) of the conflict graph. At this time, for example, as illustrated in FIG. 11, the same atom types in the molecules A and B are combined and employed as nodes of the conflict graph. In the example illustrated in FIG. 11, combinations of A1, B1, and B4 that represent the atom type "c3", a combination of A2 and B2 that represent the atom type "c2", and

a combination of A5 and B5 that represent the atom type "o" are employed as nodes of the conflict graph. In this manner, by employing, as a node, the combination of not the same elements but the atoms that have the same atom type, which is subdivided more finely than the elemental species, the number of nodes may be suppressed, and the number of bits of a calculator to be used to solve the maximum independent set problem may be made smaller.

In the example in FIG. 11, the number of nodes of the conflict graph created from the molecules A and B expressed as graphs is given as four, as illustrated in FIG. 11.

On the other hand, in the example in FIG. 2, six nodes are created by combining the carbons of the molecule A and the carbons of the molecule B, and four nodes are created by combining the oxygens of the molecule A and the oxygens of the molecule B. Therefore, the number of nodes of the conflict graph created from the molecules A and B expressed as graphs is given as ten.

Subsequently, a conflict graph is created, and is given as illustrated in FIG. 12.

In an example of the technology disclosed in the present application, for example, the first material denotes a material to be compared with the second material for which the similarity is to be worked out.

The first material is not particularly limited and can be appropriately selected according to the purpose, which may be a molecule or may not be a molecule. Examples of the first material other than molecules include inorganic crystals or the like.

Furthermore, the first material is not particularly limited as long as a material that can be expressed as a graph is employed, and can be appropriately selected according to the purpose.

In the example of the technology disclosed in the present application, for example, the second material means a target material for which the similarity to the first material is to be worked out.

The second material is not particularly limited and can be appropriately selected according to the purpose, which may be a molecule or may not be a molecule. Examples of the second material other than molecules include inorganic crystals, or the like.

Furthermore, the second material is not particularly limited as long as a material that can be expressed as a graph is employed, and can be appropriately selected according to the purpose.

Here, in the example of the technology disclosed in the present application, it is preferable that the chemical structure data of the first material and the second material be input as a chemical structure data group (database) containing a large number of materials. For example, it is preferable that the similarity calculation device as an example of the technology disclosed in the present application have a chemical structure data group containing a large number of materials.

The format (data structure) of the chemical structure data group is not particularly limited and can be appropriately selected according to the purpose; examples of the format include the SDF format described earlier, or the like.

In the example of the technology disclosed in the present application, for example, the structure of each of the first material and the second material may be specified by accepting the compound names or common names or the like of the first material and the second material, and collating the first material and the second material with the chemical structure data group. Furthermore, in the example of the technology disclosed in the present application, for example, the structures of the first material and the second material may be specified by directly inputting the chemical structure data of the first material and the second material.

In the example of the technology disclosed in the present application, for example, when the similarity between the first material and the second material is worked out using above Formulas (1) and (2), parameters of above Formulas (1) and (2) are appropriately optimized.

In the example of the technology disclosed in the present application, for example, as in the above-described prior art, the similarity can be worked out using Formula (1), by searching for the maximum independent set based on the molecular structures of the first material and the second material.

[Mathematical Formula 6]

$$H = -\alpha \sum_{i=0}^{n-1} b_i x_i + \beta \sum_{i,j=0}^{n-1} w_{ij} x_i x_j \quad \text{... Formula (1)}$$

However, in above Formula (1), H denotes a Hamiltonian in which minimizing H means searching for the maximum independent set.

The sign n is understood as the number of nodes in the conflict graph of the first material and the second material expressed as graphs.

Furthermore, the conflict graph is understood as a graph that employs, as nodes, combinations of respective node atoms that constitute the first material expressed as a graph and respective node atoms that constitute the second

material expressed as a graph, and that is created based on the rule that an edge is created between two nodes when the nodes are compared and are not identical to each other, and no edge is created between two nodes when the nodes are compared and are identical to each other.

The sign bi denotes a numerical value that represents a bias for the i-th node.

The sign $w_{ij}$ has a positive non-zero number when there is an edge between the i-th node and a j-th node, and has zero when there is no edge between the i-th node and the j-th node.

The sign $x_i$ denotes a binary variable that represents that the i-th node has 0 or 1, and the sign $x_j$ denotes a binary variable that represents that the j-th node has 0 or 1.

Note that $\alpha$ and $\beta$ denote positive numbers.

Here, in the example of the technology disclosed in the present application, the case where "two nodes are compared and are identical to each other" means that, when two nodes are compared, these nodes are constituted by node atoms in identical situations (bonding situations) to each other. Likewise, in the example of the technology disclosed in the present application, the case where "two nodes are compared and are not identical to each other" means that, when a plurality of nodes is compared, these nodes are constituted by node atoms in different situations (bonding situations) from each other.

Here, the bonding situation may be denoted by the bond order, but may be denoted by a bonding situation that is more detailed than the bond order. For example, the bonding situation may include whether or not the concerned combination is included in an aromatic ring and whether or not the concerned combination has a covalent, ionic or coordinate bond. Examples of the bonding situation that is more detailed than the bond order include a bond type defined by Austin model 1 (AM1)-bond charge correction (BCC).

The bond type defined by AM1-bond charge correction (BCC) is introduced in the following document, for example.

Document: JAKALIAN, ARAZ; JACK, DAVID B.; BAYLY, CHRISTOPHER I., "Fast, Efficient Generation of High-Quality Atomic Charges. AM1-BCC Model: II. Parameterization and Validation", Journal of Computational Chemistry, 23: 1623-1641, 2002

In the example of the technology disclosed in the present application, when a search for the maximum independent set is performed using above Formula (1), it is not highly prioritized to create the conflict graph of the first material and second material expressed as graphs, and it suffices that at least above Formula (1) can be minimized. For example, in the example of the technology disclosed in the present application, the search for the maximum independent set in the conflict graph of the first material and the second material is replaced with a combination optimization problem in a Hamiltonian in which minimizing means the searching for the maximum independent set, and solved. Here, the minimization of the Hamiltonian represented by the Ising model equation in the QUBO format as in above Formula (1) can be executed in a short time by performing the annealing method (annealing) using an annealing machine or the like. Note that details of the annealing method will be described later.

Furthermore, in the example of the technology disclosed in the present application, for example, as in the above-described prior art, the similarity can be worked out based on the retrieved maximum independent set using Formula (2) .

[Mathematical Formula 7]

$$S(G_A, G_B) = \delta \max\left\{\frac{|V_C^A|}{|V_A|}, \frac{|V_C^B|}{|V_B|}\right\} + (1-\delta)\min\left\{\frac{|V_C^A|}{|V_A|}, \frac{|V_C^B|}{|V_B|}\right\} \quad \text{... Formula (2)}$$

However, in above Formula (2), $G_A$ represents the first material expressed as a graph, and $G_B$ represents the second material expressed as a graph; $S(G_A, G_B)$ represents the similarity between the first material expressed as a graph and the second material expressed as a graph, is represented as 0 to 1, and means that the closer to 1, the higher the similarity.

Furthermore, $V_A$ represents the total number of node atoms of the first material expressed as a graph, and $V_c^A$ represents the number of node atoms included in the maximum independent set of the conflict graph among the node atoms of the first material expressed as a graph.

$V_B$ represents the total number of node atoms of the second material expressed as a graph, and $V_c^B$ represents the number of node atoms included in the maximum independent set of the conflict graph among the node atoms of the second material expressed as a graph.

Note that $\delta$ denotes a number from 0 to 1.

An exemplary sequence from reading the molecular structure to searching for a maximum independent set will be further described using acetic acid and methyl acetate as examples.

First, the chemical structures of acetic acid (A) and methyl acetate (B) illustrated in FIG. 13 are read from a file

format such as SDF.

Next, using the read chemical structure as an input, the atom type and bond type (bonding situation) are defined using antechamber. Here, antechamber is a module included in AMBER Tool.

As a consequence, the atom type and bond type (bonding situation) of each of acetic acid (A) and methyl acetate (B) are defined as follows. Note that the numbers below correspond to the numbers allocated to the atoms of the molecules in FIG. 13.

(I) Atom Type

(A) 1: c3

2: c2
3: oh
5: o

(B) 1: c3

2: c2
3: os
4: c3
5: o

(II) Bond Type

(A) 1-2: Single Bond

2-3: Single Bond
2-5: Double Bond

(B) 1-2: Single Bond

2-3: Single Bond
2-5: Double Bond
3-4: Single Bond

Then, the atom type and bond type are employed as a node label and an edge label, respectively, and expressed as graphs, which are given as illustrated in FIG. 14.

Next, using the created graphs, a pair of the same atom types is found in accordance with the flowchart illustrated in FIG. 15, and the found pair is employed as a node of the conflict graph. Here, the meanings of the reference signs in the flowchart illustrated in FIG. 15 are as follows.

- ia: atom index of molecule A (acetic acid)
- ja: atom index of molecule B (methyl acetate)
- nA: number of all atoms of molecule A (acetic acid)
- nB: number of all atoms of molecule B (methyl acetate)
- at[i]: atom type of atom i

As a result, the four pairs illustrated in FIG. 16 are employed as nodes of the conflict graph. Then, one bit is allocated to each node.

Next, an edge is created between nodes with different bonding situations.

FIG. 17 illustrates the conflict graph. Note that in the conflict graph in FIG. 17, solid lines between nodes represent edges, and broken lines between nodes represent that no edges have been created.

Then, in accordance with the flow illustrated in FIG. 18, a weight between nodes (bits) without edges is designated as 0, and a weight between nodes (bits) with edges is designated as 1 (or an integer value equal to or greater than 1).

Here, for example, regarding [0]-[1], $w_{01}$ is given as 0 because A1-A2 is a single bond and B1-B2 is a single bond. Regarding [0]-[2], A1-A1 is a self-bond, and there is no bond for B1-B4. This means, for example, that [0]-[2] is deemed as nodes that are not identical to each other. Therefore, $w_{02}$ is given as 1. Regarding [1]-[2], $w_{12}$ is given as 1 because A2-A1 is a single bond and B2-B4 has no direct bond.

Next, using Formula (1) described above, a search for the maximum independent set, which is in a bit state that minimizes the Hamiltonian (H), is performed. The search for the maximum independent set is performed using, for example, Digital Annealer (registered trademark).

As a result, as illustrated in FIG. 19, it can be seen that the maximum independent set is taken when $x_0$[A1B1] = 1, $x_1$[A2B2] = 1, $x_2$[A1B4] = 0, and $x_3$[A5B5] = 1 are satisfied. Then, the maximum common substructure of acetic acid and methyl acetate at that time is as illustrated in FIG. 19.

Hereinafter, the example of the technology disclosed in the present application will be described in more detail using exemplary device configurations, flowcharts, and the like.

FIG. 20 illustrates an exemplary hardware configuration of the similarity calculation device disclosed in the present application.

In the similarity calculation device 10, for example, a control unit 11, a memory 12, a storage unit 13, a display unit 14, an input unit 15, an output unit 16, and an input/output (I/O) interface unit 17 are connected to each other via a system bus 18.

The control unit 11 performs arithmetic operations (for example, four arithmetic operations, comparison operations, and arithmetic operations for the annealing method), hardware and software operation control, and the like.

The control unit 11 is not particularly limited and can be appropriately selected according to the purpose; for example, the control unit 11 may be a central processing unit (CPU) or an optimizing device used for the annealing method described later, or may be a combination of these pieces of equipment.

The creation unit, the search unit, and the computation unit of the similarity calculation device disclosed in the present application can be achieved by the control unit 11, for example.

The memory 12 is a memory such as a random access memory (RAM) or a read only memory (ROM). The RAM stores an operating system (OS), an application program, and the like read from the ROM and the storage unit 13, and functions as a main memory and a work area of the control unit 11.

The storage unit 13 is a device that stores various kinds of programs and data, and may be a hard disk, for example. The storage unit 13 stores a program to be executed by the control unit 11, data to be used in executing the program, an OS, and the like.

Furthermore, a program disclosed in the present application is stored in, for example, the storage unit 13, is loaded into the RAM (main memory) of the memory 12, and is executed by the control unit 11.

The display unit 14 is a display device, and may be a display device such as a cathode ray tube (CRT) monitor or a liquid crystal panel, for example.

The input unit 15 is an input device for various kinds of data, and may be a keyboard or a pointing device (such as a mouse or the like), for example.

The output unit 16 is an output device for various kinds of data, and may be a printer or the like, for example.

The I/O interface unit 17 is an interface for connecting various external devices.

The I/O interface unit 17 enables input and output of data on, for example, a compact disc read only memory (CD-ROM), a digital versatile disk read only memory (DVD-ROM), a magneto-optical (MO) disk, or a universal serial bus (USB) memory (USB flash drive).

FIG. 21 illustrates another exemplary hardware configuration of the similarity calculation device disclosed in the present application.

The example illustrated in FIG. 21 is an example of a case where the similarity calculation device of a cloud type is employed, and the control unit 11 is independent of the storage unit 13 and the like. In the example illustrated in FIG. 21, a computer 30 that includes the storage unit 13 and the like is connected to a computer 40 that includes the control unit 11 via network interface units 19 and 20.

The network interface units 19 and 20 are hardware that performs communication using the Internet.

FIG. 22 illustrates another exemplary hardware configuration of the similarity calculation device disclosed in the present application.

The example illustrated in FIG. 22 is an example of a case where the similarity calculation device of a cloud type is employed, and the storage unit 13 is independent of the control unit 11 and the like. In the example illustrated in FIG. 22, a computer 30 that includes the control unit 11 and the like is connected to a computer 40 that includes the storage unit 13 via network interface units 19 and 20.

FIG. 23 illustrates another exemplary hardware configuration of the similarity calculation device disclosed in the present application.

The example illustrated in FIG. 23 is an example of a case where an optimizing device 21 is included separately from the control unit 11. Furthermore, the example illustrated in FIG. 23 is an example of a case where the similarity calculation device of a cloud type is employed. In FIG. 23, the optimizing device 21 is independent of the control unit 11, the memory 12, the storage unit 13, and the like. In the example illustrated in FIG. 23, a computer 30 that includes the control unit 11 and the like is connected to a computer 40 that includes the optimizing device 21 via network interface units 19 and 20. The optimizing device 21 is, for example, an optimizing device used in the annealing method described

later.

In the example illustrated in FIG. 23, for example, the creation unit and the computation unit of the similarity calculation device disclosed in the present application are achieved by the control unit 11, and the search unit is achieved by the optimizing device 21.

FIG. 24 illustrates an exemplary functional configuration as an embodiment of the similarity calculation device disclosed in the present application. Furthermore, FIG. 25 illustrates a flowchart of an embodiment of similarity calculation disclosed in the present application.

As illustrated in FIG. 24, the similarity calculation device 10 includes a structure acquisition unit 51, a chemical structure graphing unit 52, a creation unit 53, a search unit 54, and a computation unit 55.

The structure acquisition unit 51 reads chemical structure data 60 of materials (the first material and the second material) as an input from a file format such as SDF (process: S1).

The chemical structure graphing unit 52 expresses the first material and the second material as graphs in regard to the read chemical structure data 60 (process: S2). In the created graphs, atoms that constitute nodes are classified according to the atom type, as illustrated in FIG. 10, for example.

The creation unit 53 creates a conflict graph using the created graphs (process: S3).

The search unit 54 searches for a maximum independent set in the conflict graph by executing a ground state search using the annealing method (process: S4). For example, using an annealing machine, which is an optimizing device, the maximum independent set is searched for by minimizing the Hamiltonian of Formula (1).

The computation unit 55 computes the similarity between the first material and the second material based on the maximum independent set (process: S5). For example, the similarity is computed from Formula (2).

The computed similarity is output.

The annealing machine is not particularly limited as long as a computer that adopts an annealing approach that performs a ground state search for an energy function represented by an Ising model is employed, and can be appropriately selected according to the purpose. Examples of the annealing machine include a quantum annealing machine, a semiconductor annealing machine using a semiconductor technology, and a machine that performs simulated annealing executed by software using a CPU or a graphics processing unit (GPU). Furthermore, for example, Digital Annealer (registered trademark) may be used as the annealing machine.

Examples of the annealing method and the annealing machine will be described below.

The annealing method is a method of probabilistically working out a solution using superposition of random number values and quantum bits. The following describes a problem of minimizing a value of an evaluation function to be optimized as an example. The value of the evaluation function is referred to as energy. Furthermore, when the value of the evaluation function is maximized, the sign of the evaluation function only needs to be changed.

First, a process is started from an initial state in which one of discrete values is assigned to each variable. With respect to a current state (combination of variable values), a state close to the current state (for example, a state in which only one variable is changed) is selected, and a state transition therebetween is considered. An energy change with respect to the state transition is calculated. Depending on the value, it is probabilistically determined whether to adopt the state transition to change the state or not to adopt the state transition to keep the original state. In a case where an adoption probability when the energy goes down is selected to be larger than that when the energy goes up, it can be expected that a state change will occur in a direction that the energy goes down on average, and that a state transition will occur to a more appropriate state over time. Then, there is a possibility that an optimum solution or an approximate solution that gives energy close to the optimum value can be obtained finally.

If this is adopted when the energy goes down deterministically and is not adopted when the energy goes up, the energy change decreases monotonically in a broad sense with respect to time, but no further change occurs when a local solution is reached. As described above, since there are a very a large number of local solutions in the discrete optimization problem, a state is almost certainly caught in a local solution that is not so close to an optimum value. Therefore, when the discrete optimization problem is solved, it is important to determine probabilistically whether to adopt the state.

In the annealing method, it has been proved that by determining an adoption (permissible) probability of a state transition as follows, a state reaches an optimum solution in the limit of infinite time (iteration count).

In the following, a method of working out an optimum solution using the annealing method will be described step by step.

(1) For an energy change (energy reduction) value ($-\Delta E$) due to a state transition, a permissible probability p of the state transition is determined by any one of the following functions f().

[Mathematical Formula 8]

$$p(\Delta E, T) = f(-\Delta E / T)$$ (Formula 1-1)

[Mathematical Formula 9]

$$f_{metro}(x) = \min(1, e^x)$$ (Metropolis Method) (Formula 1-2)

[Mathematical Formula 10]

$$f_{Gibbs}(x) = \frac{1}{1+e^{-x}}$$ (Gibbs Method) (Formula 1-3)

Here, T denotes a parameter called a temperature value and can be changed as follows, for example.

(2) The temperature value T is logarithmically reduced with respect to an iteration count t as represented by the following Formula.

[Mathematical Formula 11]

$$T = \frac{T_0 \log(c)}{\log(t+c)}$$ Formula (2)

Here, $T_0$ is an initial temperature value, and is desirably a sufficiently large value depending on a problem.

In a case where the permissible probability represented by the Formula in (1) is used, if a steady state is reached after sufficient iterations, an occupation probability of each state follows a Boltzmann distribution for a thermal equilibrium state in thermodynamics.

Then, when the temperature is gradually lowered from a high temperature, an occupation probability of a low energy state increases. Therefore, it is considered that the low energy state is obtained when the temperature is sufficiently lowered. Since this state is very similar to a state change caused when a material is annealed, this method is referred to as the annealing method (or pseudo-annealing method). Note that probabilistic occurrence of a state transition that increases energy corresponds to thermal excitation in physics.

FIG. 26 illustrates an exemplary functional configuration of an optimizing device that performs the annealing method. However, in the following description, a case of generating a plurality of state transition candidates is also described, but a basic annealing method generates one transition candidate at a time.

An optimizing device 100 includes a state holding unit 111 that holds a current state S (a plurality of state variable values). Furthermore, the optimizing device 100 includes an energy calculation unit 112 that calculates an energy change value {-ΔEi} of each state transition when a state transition from the current state S occurs due to a change in any one of the plurality of state variable values. Moreover, the optimizing device 100 includes a temperature control unit 113 that controls the temperature value T and a transition control unit 114 that controls a state change.

The transition control unit 114 probabilistically determines whether to accept or not any one of a plurality of state transitions according to a relative relationship between the energy change value {-ΔEi} and thermal excitation energy, based on the temperature value T, the energy change value {-ΔEi}, and a random number value.

Here, the transition control unit 114 includes a candidate generation unit 114a that generates a state transition candidate, and a propriety determination unit 114b for probabilistically determining whether or not to permit a state transition for each candidate on the basis of the energy change value {-ΔEi} and the temperature value T. Moreover, the transition control unit 114 includes a transition determination unit 114c that determines a candidate to be adopted from the candidates that have been permitted, and a random number generation unit 114d that generates a random variable.

The operation of the optimizing device 100 in one iteration is as follows.

First, the candidate generation unit 114a generates one or more state transition candidates (candidate number {Ni}) from the current state S held in the state holding unit 111 to a next state. Next, the energy calculation unit 112 calculates the energy change value {-ΔEi} for each state transition listed as a candidate using the current state S and

the state transition candidates. The propriety determination unit 114b permits a state transition with a permissible probability of the Formula in above (1) according to the energy change value {-ΔEi} of each state transition using the temperature value T generated by the temperature control unit 113 and the random variable (random number value) generated by the random number generation unit 114d.

Then, the propriety determination unit 114b outputs propriety {fi} of each state transition. In a case where there is a plurality of permitted state transitions, the transition determination unit 114c randomly selects one of the permitted state transitions using a random number value. Then, the transition determination unit 114c outputs a transition number N and transition propriety f of the selected state transition. In a case where there is a permitted state transition, a state variable value stored in the state holding unit 111 is updated according to the adopted state transition.

Starting from an initial state, the above-described iteration is repeated while the temperature value is lowered by the temperature control unit 113. When a completion determination condition such as reaching a certain iteration count or energy falling below a certain value is satisfied, the operation is completed. An answer output by the optimizing device 100 is a state when the operation is completed.

FIG. 27 is a circuit-level block diagram of an exemplary configuration of the transition control unit in a normal annealing method for generating one candidate at a time, particularly an arithmetic unit for the propriety determination unit.

The transition control unit 114 includes a random number generation circuit 114b1, a selector 114b2, a noise table 114b3, a multiplier 114b4, and a comparator 114b5.

The selector 114b2 selects and outputs a value corresponding to the transition number N, which is a random number value generated by the random number generation circuit 114b1, among energy change values {-ΔEi} calculated for respective state transition candidates.

The function of the noise table 114b3 will be described later. For example, a memory such as a RAM or a flash memory can be used as the noise table 114b3.

The multiplier 114b4 outputs a product obtained by multiplying a value output by the noise table 114b3 by the temperature value T (corresponding to the above-described thermal excitation energy).

The comparator 114b5 outputs a comparison result obtained by comparing a multiplication result output by the multiplier 114b4 with -ΔE, which is an energy change value selected by the selector 114b2, as transition propriety f.

The transition control unit 114 illustrated in FIG. 27 basically implements the above-described functions as they are. However, a mechanism that permits a state transition with a permissible probability represented by the Formula in (1) will be described in more detail.

A circuit that outputs 1 at a permissible probability p and outputs 0 at a permissible probability (1-p) can be achieved by inputting a uniform random number that takes the permissible probability p for input A and takes a value of an interval [0, 1) for input B in a comparator that has two inputs A and B, outputs 1 when A > B is satisfied and outputs 0 when A < B is satisfied. Therefore, if the value of the permissible probability p calculated on the basis of the energy change value and the temperature value T using the Formula in (1) is input to input A of this comparator, the above-described function can be achieved.

This means that, with a circuit that outputs 1 when f(ΔE/T) is larger than u, in which f is a function used in the Formula in (1), and u is a uniform random number that takes a value of the interval [0, 1), the above-described function can be achieved.

Furthermore, the same function as the above-described function can also be achieved by making the following modification.

Applying the same monotonically increasing function to two numbers does not change the magnitude relationship. Therefore, an output is not changed even if the same monotonically increasing function is applied to two inputs of the comparator. If an inverse function $f^{-1}$ of f is adopted as this monotonically increasing function, it can be seen that a circuit that outputs 1 when -ΔE/T is larger than $f^{-1}(u)$ can be given. Moreover, since the temperature value T is positive, it can be seen that a circuit that outputs 1 when -ΔE is larger than $Tf^{-1}(u)$ may be sufficient.

The noise table 114b3 in FIG. 27 is a conversion table for achieving this inverse function $f^{-1}(u)$, and is a table that outputs a value of the following function to an input that discretizes the interval [0,1).

[Mathematical Formula 12]

$$f_{metro}^{-1}(u) = \log(u) \quad \text{(Formula 3-1)}$$

[Mathematical Formula 13]

$$f_{Gibbs}^{-1}(u) = \log\left(\frac{u}{1-u}\right) \quad \text{(Formula 3-2)}$$

The transition control unit 114 also includes a latch that holds a determination result and the like, a state machine that generates a timing thereof, and the like, but these are not illustrated in FIG. 27 for simplicity of illustration.

FIG. 28 is a diagram illustrating an exemplary operation flow of the transition control unit 114. The operation flow illustrated in FIG. 28 includes a step of selecting one state transition as a candidate (S0001), a step of determining propriety of the state transition by comparing an energy change value for the state transition with a product of a temperature value and a random number value (S0002), and a step of adopting the state transition if the state transition is permitted, and not adopting the state transition if the state transition is not permitted (S0003).

The program disclosed in the present application can be configured as, for example, a program that causes a computer to execute the similarity calculation method disclosed in the present application. Furthermore, a suitable mode of the program disclosed in the present application can be made the same as the suitable mode of the similarity calculation method disclosed in the present application, for example.

The program disclosed in the present application can be created using various known programming languages according to the configuration of a computer system to be used, the type and version of the operating system, and the like.

The program disclosed in the present application may be recorded in a recording medium such as an internal hard disk or an external hard disk, or may be recorded in a recording medium such as a CD-ROM, DVD-ROM, MO disk, or USB memory.

Moreover, in a case where the program disclosed in the present application is recorded in a recording medium as mentioned above, the program can be directly used, or can be installed into a hard disk and then used through a recording medium reader included in the computer system, depending on the situation. Furthermore, the program disclosed in the present application may be recorded in an external storage area (another computer or the like) accessible from the computer system through an information communication network. In this case, the program disclosed in the present application, which is recorded in an external storage area, can be used directly, or can be installed in a hard disk and then used from the external storage area through the information communication network, depending on the situation.

Note that the program disclosed in the present application may be divided for each of any pieces of processing, and recorded in a plurality of recording media.

(Recording Medium)

A recording medium disclosed in the present application is obtained by recording the program disclosed in the present application.

The recording medium disclosed in the present application is computer-readable.

The recording medium disclosed in the present application is not particularly limited, and can be appropriately selected according to the purpose. Examples of the recording medium include an internal hard disk, an external hard disk, a CD-ROM, a DVD-ROM, an MO disk, and a USB memory.

Furthermore, the recording medium disclosed in the present application may include a plurality of recording media in which the program disclosed in the present application is recorded after being divided for each of any pieces of processing.

The recording medium disclosed in the present application may be transitory or non-transitory.

<Calculation Examples>

As one calculation example of the similarity calculation device disclosed in the present application, the similarity between linalool and fragrance molecules was calculated.

Linalool has the chemical structure illustrated in FIG. 29 and has a citrus scent.

As fragrance molecules, among the molecules listed in Table 1 of the Food Sanitation Law Enforcement Regulations, 132 molecules whose scent is registered in The Good Scents Company Information System (http://www.thegoodscentscompany.com/index.html) were used.

<<Conventional Example>>

The similarity was calculated in accordance with the flow illustrated in FIG. 25.

The chemical structure data of the fragrance molecules was read from the SDF file format as an input (process:

S1).

The read chemical structure data was expressed as graphs (process: S2). In the created graphs, the atoms that constitute nodes are classified according to the elemental species.

A conflict graph was created using the created graphs (process: S3). Here, when the conflict graph was created, nodes of the conflict graph were created from combinations of two atoms that are the same elemental species between two molecules.

The maximum independent set in the conflict graph was searched for by executing a ground state search using the annealing method (process: S4). Here, using an annealing machine, which is an optimizing device, the maximum independent set was searched for by minimizing the Hamiltonian of Formula (1).

The similarity was computed based on the maximum independent set (process: S6). Here, the similarity was computed from Formula (2).

In the conventional example, when the conflict graph of linalool and terpineol was created, 101 nodes were created. This means that, as illustrated in FIG. 30, 101 bits were taken to search for the maximum independent set.

Furthermore, Table 1 illustrates the result of calculating the similarity to linalool for a part of the 132 molecules according to the conventional example.

[Table 1]

| Molecule Name | Scent (Odor) | Structural Similarity |
|---|---|---|
| Linalool | citrus floral sweet boise de rose woody green blueberry | 1.00 |
| Terpineol | pine terpene lilac citrus woody floral | 0.91 |
| Linalyl Acetate | sweet green citrus bergamot lavender woody | 0.89 |
| Citronellal | clean herbal citrus | 0.82 |
| Geraniol | sweet floral fruity rose waxy citrus | 0.82 |
| Citronellol | floral leather waxy rose bud citrus | 0.82 |
| Citral | citrus lemon | 0.82 |
| Menthol | peppermint cool woody | 0.82 |
| Terpinyl Acetate | herbal bergamot lavender lime citrus | 0.81 |

<<Example>>

The similarity was calculated in accordance with the flow illustrated in FIG. 25.

The chemical structure data of the fragrance molecules was read from the SDF file format as an input (process: S1).

The read chemical structure data was expressed as graphs (process: S2). In the created graphs, the atoms that constitute nodes are classified according to the atom type of general AMBER force field (GAFF).

A conflict graph was created using the created graphs (process: S3). Here, when the conflict graph was created, nodes of the conflict graph were created from combinations of two atoms that have the same GAFF atom type between two molecules.

The maximum independent set in the conflict graph was searched for by executing a ground state search using the annealing method (process: S4). Here, using an annealing machine, which is an optimizing device, the maximum independent set was searched for by minimizing the Hamiltonian of Formula (1).

The similarity was computed based on the maximum independent set (process: S6). Here, the similarity was computed from Formula (2).

In the example, when the conflict graph of linalool and terpineol was created, 57 nodes were created. This means that, as illustrated in FIG. 31, 57 bits were taken to search for the maximum independent set.

Furthermore, Table 2 illustrates the result of calculating the similarity to linalool for a part of the 132 molecules according to the example.

[Table 2]

| Molecule Name | Scent (Odor) | Structural Similarity |
|---|---|---|
| Linalool | citrus floral sweet boise de rose woody green blueberry | 1.00 |

(continued)

| Molecule Name | Scent (Odor) | Structural Similarity |
|---|---|---|
| Terpineol | pine terpene lilac citrus woody floral | 0.82 |
| Citronellal | clean herbal citrus | 0.82 |
| Geraniol | sweet floral fruity rose waxy citrus | 0.82 |
| Linalyl Acetate | | 0.81 |
| Terpinyl Acetate | herbal bergamot lavender lime citrus | 0.73 |
| Citronellol | floral leather waxy rose bud citrus | 0.73 |
| Citral | citrus lemon | 0.73 |
| Menthol | peppermint cool woody | 0.64 |

Comparing Table 1 and Table 2, in the example, the similarity of menthol, which is not citrus-based, indicated a lower value than the value of the similarity computed in the conventional example. This means that the example has a higher accuracy of the similarity than the accuracy of the conventional example. The cause of this difference is considered that, in the method of the example, the substructure ($H_3C$-CH) and the substructure ($H_3C$-$CH_2$) in the following two structures are not identically treated, while in the conventional example, the substructure ($H_3C$-CH) and the substructure ($H_3C$-$CH_2$) in the following two structures are identically treated.

[Chemical Formula 1]

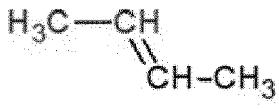

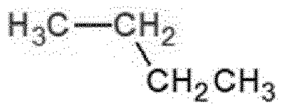

**Claims**

1. A similarity calculation device that calculates a similarity between a first material and a second material, the similarity calculation device comprising:

    a creation unit that creates a conflict graph that is a graph that has a plurality of nodes made up of combinations of respective atoms that constitute the first material and respective atoms that constitute the second material, and an edge formed between two nodes among the plurality of nodes, and that has an edge between two nodes when the nodes are compared and are not identical to each other, and has no edge between two nodes when the nodes are compared and are identical to each other;
    a search unit that searches for a maximum independent set in the conflict graph by executing a ground state search using an annealing method; and
    a computation unit that computes the similarity between the first material and the second material based on the maximum independent set, wherein
    the plurality of nodes of the conflict graph is each made up of a combination of two atoms that have an atom type that is same between the first material and the second material, the atom type being subdivided more finely than elemental species.

2. The similarity calculation device according to claim 1, wherein the atom type includes a type of orbital hybridization, a type of aromaticity, or a type of chemical environment of an atom, or any combination of the type of hybrid orbital of an outermost shell electron, the type of aromaticity, or the type of orbital hybridization.

3. The similarity calculation device according to claim 1 or 2, wherein the plurality of nodes of the conflict graph is each

made up of a combination of two atoms that are same in the atom type and bond type between the first material and the second material.

**4.** The similarity calculation device according to claim 3, wherein the bond type includes whether the combination is included in an aromatic ring, or whether the combination has a covalent, ionic or coordinate bond, or a combination of whether the combination is included in an aromatic ring, or whether the combination has a covalent, ionic or coordinate bond.

**5.** The similarity calculation device according to any one of claims 1 to 4, wherein the search unit uses following Formula (1) to search for the maximum independent set based on molecular structures of the first material and the second material:
[Mathematical Formula 1]

$$H = -\alpha \sum_{i=0}^{n-1} b_i x_i + \beta \sum_{i,j=0}^{n-1} w_{ij} x_i x_j \quad \text{... Formula (1)}$$

in above Formula (1),

the H denotes a Hamiltonian in which minimizing the H means searching for the maximum independent set,
the n is understood as a number of nodes in the conflict graph of the first material and the second material expressed as graphs,
the bi denotes a numerical value that represents a bias for an i-th node among the nodes,
the $w_{ij}$ has
a positive non-zero number when there is an edge between the i-th node and a j-th node among the nodes, and zero when there is no edge between the i-th node and the j-th node,
the $x_i$ denotes a binary variable that represents that the i-th node has 0 or 1,
the $x_j$ denotes a binary variable that represents that the j-th node has 0 or 1, and
the $\alpha$ and the $\beta$ denote positive numbers.

**6.** The similarity calculation device according to any one of claims 1 to 5, wherein the computation unit uses following Formula (2) to work out the similarity based on the retrieved maximum independent set:
[Mathematical Formula 2]

$$S(G_A, G_B) = \delta \max\left\{\frac{|V_C^A|}{|V_A|}, \frac{|V_C^B|}{|V_B|}\right\} + (1-\delta) \min\left\{\frac{|V_C^A|}{|V_A|}, \frac{|V_C^B|}{|V_B|}\right\} \quad \text{... Formula (2)}$$

in above Formula (2),

the $G_A$ represents the first material expressed as a graph,
the $G_B$ represents the second material expressed as a graph,
the $S(G_A, G_B)$ represents the similarity between the first material expressed as the graph and the second material expressed as the graph, is represented as 0 to 1, and means that the closer to 1, the higher the similarity,
the $V_A$ represents a total number of node atoms of the first material expressed as the graph,
the $V_c^A$ represents a number of some of the node atoms included in the maximum independent set of the conflict graph among the node atoms of the first material expressed as the graph,
the $V_B$ represents a total number of node atoms of the second material expressed as the graph,
the $V_c^B$ represents a number of some of the node atoms included in the maximum independent set of the conflict graph among the node atoms of the second material expressed as the graph, and
the $\delta$ denotes a number from 0 to 1.

**7.** A similarity calculation method that calculates a similarity between a first material and a second material, the similarity calculation method comprising:

creating a conflict graph that is a graph that has a plurality of nodes made up of combinations of respective atoms that constitute the first material and respective atoms that constitute the second material, and an edge formed between two nodes among the plurality of nodes, and that has an edge between two nodes when the nodes are compared and are not identical to each other, and has no edge between two nodes when the nodes are compared and are identical to each other;
searching for a maximum independent set in the conflict graph by executing a ground state search using an annealing method; and
computing the similarity between the first material and the second material based on the maximum independent set, wherein
the plurality of nodes of the conflict graph is each made up of a combination of two atoms that have an atom type that is same between the first material and the second material, the atom type being subdivided more finely than elemental species.

8. The similarity calculation method according to claim 7, wherein the atom type includes a type of orbital hybridization, a type of aromaticity, or a type of chemical environment of an atom, or any combination of the type of orbital hybridization, the type of aromaticity, or the type of chemical environment of an atom.

9. The similarity calculation method according to claim 7 or 8, wherein the plurality of nodes of the conflict graph is each made up of a combination of two atoms that are same in the atom type and bond type between the first material and the second material.

10. The similarity calculation method according to claim 9, wherein the bond type includes whether the combination is included in an aromatic ring, or whether the combination has a covalent, ionic or coordinate bond, or a combination of whether the combination is included in an aromatic ring, or whether the combination has a covalent, ionic or coordinate bond.

11. The similarity calculation method according to any one of claims 7 to 10, wherein the search unit uses following Formula (1) to search for the maximum independent set based on molecular structures of the first material and the second material:
[Mathematical Formula 1]

$$H = -\alpha \sum_{i=0}^{n-1} b_i x_i + \beta \sum_{i,j=0}^{n-1} w_{ij} x_i x_j \quad \text{... Formula (1)}$$

in above Formula (1),

the H denotes a Hamiltonian in which minimizing the H means searching for the maximum independent set,
the n is understood as a number of nodes in the conflict graph of the first material and the second material expressed as graphs,
the bi denotes a numerical value that represents a bias for an i-th node among the nodes,
the $w_{ij}$ has
a positive non-zero number when there is an edge between the i-th node and a j-th node among the nodes, and zero when there is no edge between the i-th node and the j-th node,
the $x_i$ denotes a binary variable that represents that the i-th node has 0 or 1,
the $x_j$ denotes a binary variable that represents that the j-th node has 0 or 1, and
the $\alpha$ and the $\beta$ denote positive numbers.

12. The similarity calculation method according to any one of claims 7 to 11, wherein the computation unit uses following Formula (2) to work out the similarity based on the retrieved maximum independent set:
[Mathematical Formula 2]

$$S(G_A, G_B) = \delta \max\left\{\frac{|V_C^A|}{|V_A|}, \frac{|V_C^B|}{|V_B|}\right\} + (1-\delta) \min\left\{\frac{|V_C^A|}{|V_A|}, \frac{|V_C^B|}{|V_B|}\right\} \quad \text{... Formula (2)}$$

in above Formula (2),

the $G_A$ represents the first material expressed as a graph,
the $G_B$ represents the second material expressed as a graph,
the $S(G_A, G_B)$ represents the similarity between the first material expressed as the graph and the second material expressed as the graph, is represented as 0 to 1, and means that the closer to 1, the higher the similarity,
the $V_A$ represents a total number of node atoms of the first material expressed as the graph,
the $V_c^A$ represents a number of some of the node atoms included in the maximum independent set of the conflict graph among the node atoms of the first material expressed as the graph,
the $V_B$ represents a total number of node atoms of the second material expressed as the graph,
the $V_c^B$ represents a number of some of the node atoms included in the maximum independent set of the conflict graph among the node atoms of the second material expressed as the graph, and
the $\delta$ denotes a number from 0 to 1.

**13.** A program causing a computer to perform a creation process of:

creating a conflict graph that is a graph that has a plurality of nodes made up of combinations of respective atoms that constitute the first material and respective atoms that constitute the second material, and an edge formed between two nodes among the plurality of nodes, and that has an edge between two nodes when the nodes are compared and are not identical to each other, and has no edge between two nodes when the nodes are compared and are identical to each other;
searching for a maximum independent set in the conflict graph by executing a ground state search using an annealing method; and
computing the similarity between the first material and the second material based on the maximum independent set, wherein
the plurality of nodes of the conflict graph is each made up of a combination of two atoms that have an atom type that is same between the first material and the second material, the atom type being subdivided more finely than elemental species.

FIG. 1

MOLECULE A:
ACETIC ACID

MOLECULE B:
METHYL ACETATE

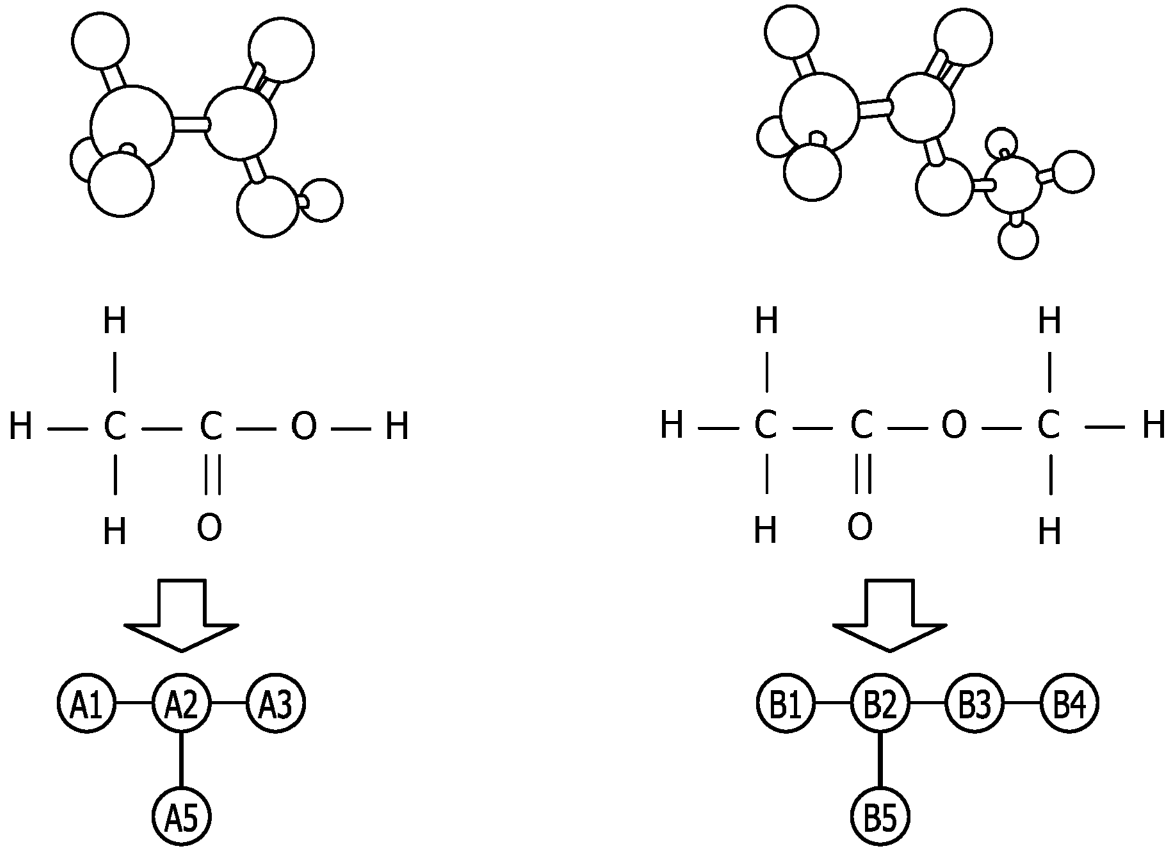

FIG. 2

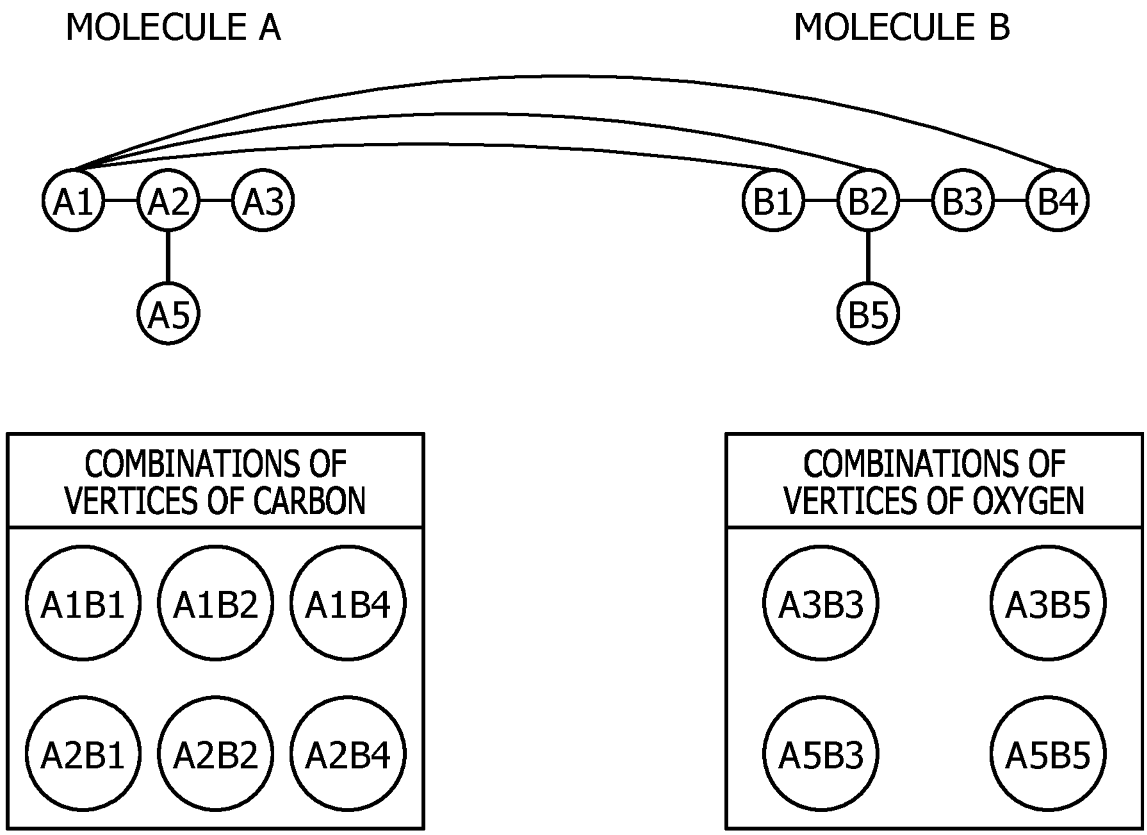
MOLECULE A
MOLECULE B
A1
A2
A3
A5
B1
B2
B3
B4
B5
COMBINATIONS OF VERTICES OF CARBON
A1B1
A1B2
A1B4
A2B1
A2B2
A2B4
COMBINATIONS OF VERTICES OF OXYGEN
A3B3
A3B5
A5B3
A5B5

FIG. 3

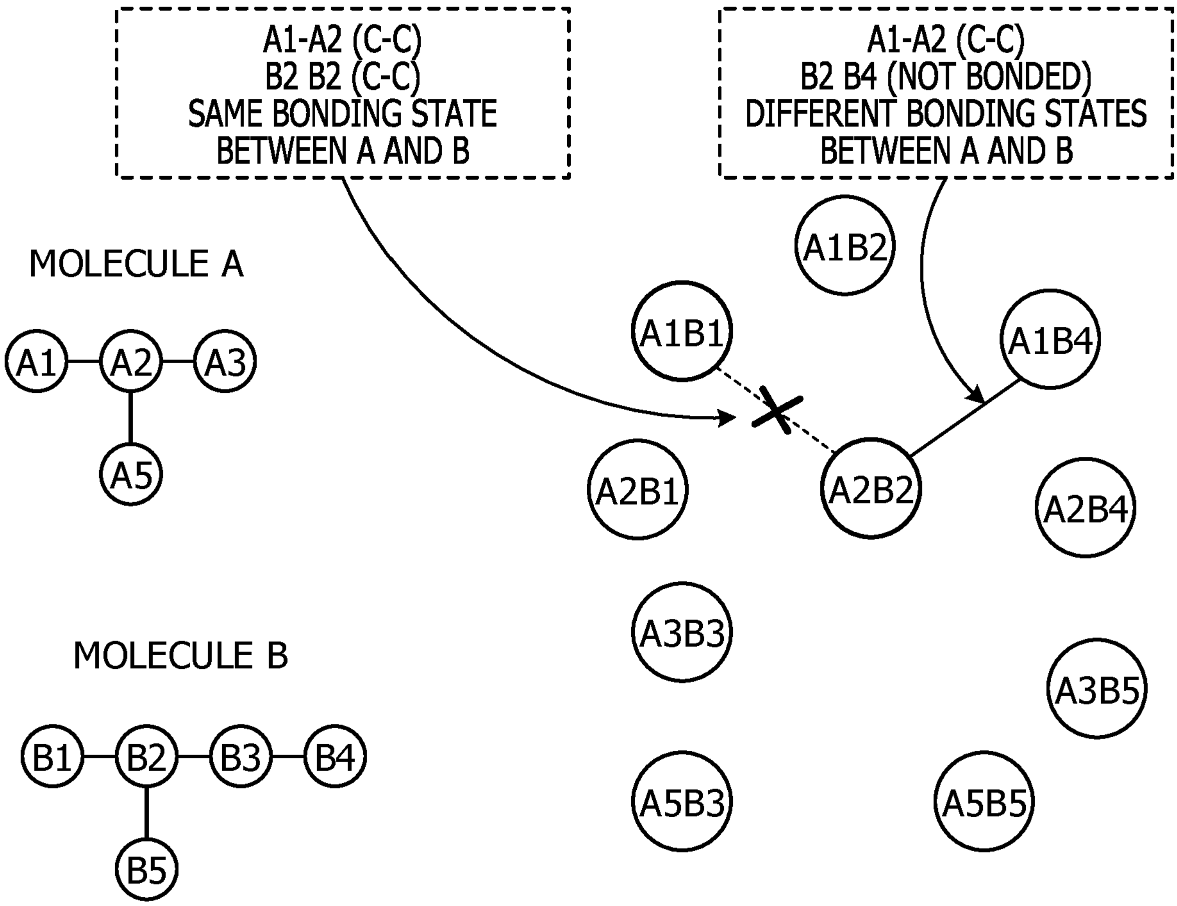
A1-A2 (C-C)
B2 B2 (C-C)
SAME BONDING STATE
BETWEEN A AND B
A1-A2 (C-C)
B2 B4 (NOT BONDED)
DIFFERENT BONDING STATES
BETWEEN A AND B
MOLECULE A
A1
A2
A3
A5
MOLECULE B
B1
B2
B3
B4
B5
A1B2
A1B1
A1B4
A2B1
A2B2
A2B4
A3B3
A3B5
A5B3
A5B5

FIG. 4

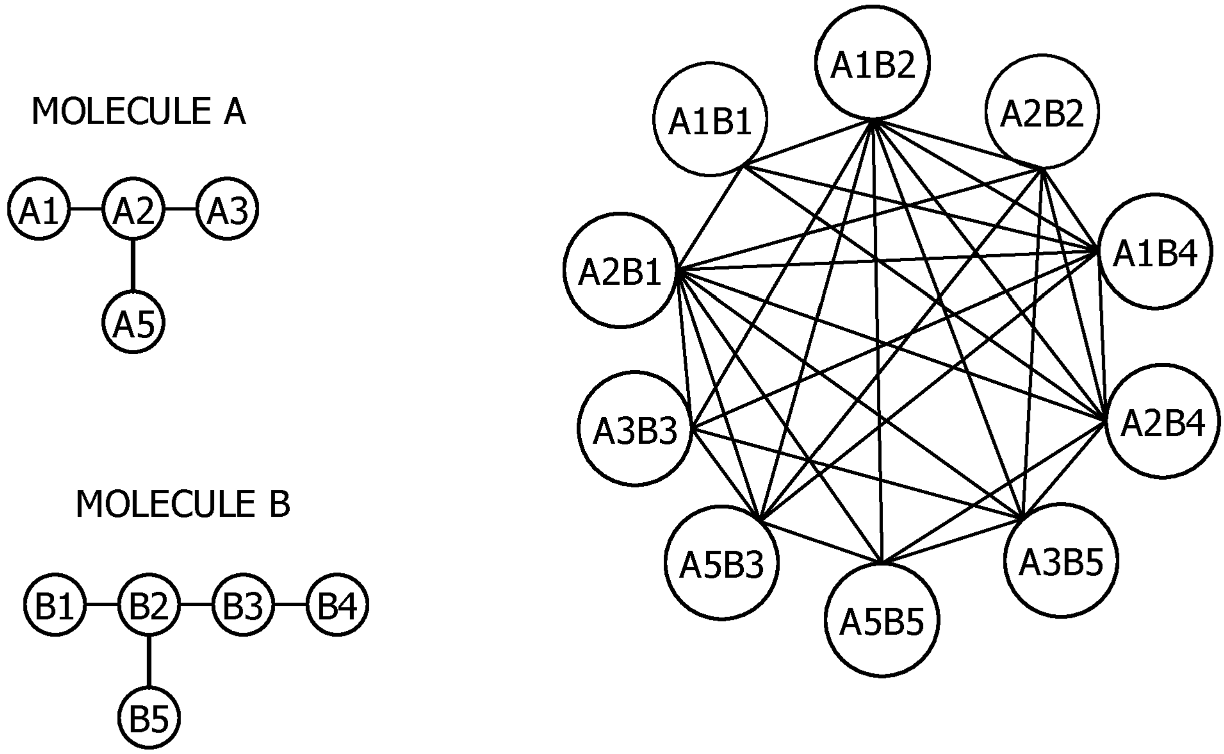
MOLECULE A
A1
A2
A3
A5
MOLECULE B
B1
B2
B3
B4
B5
A1B2
A1B1
A2B2
A1B4
A2B1
A2B4
A3B3
A5B3
A5B5
A3B5

FIG. 5

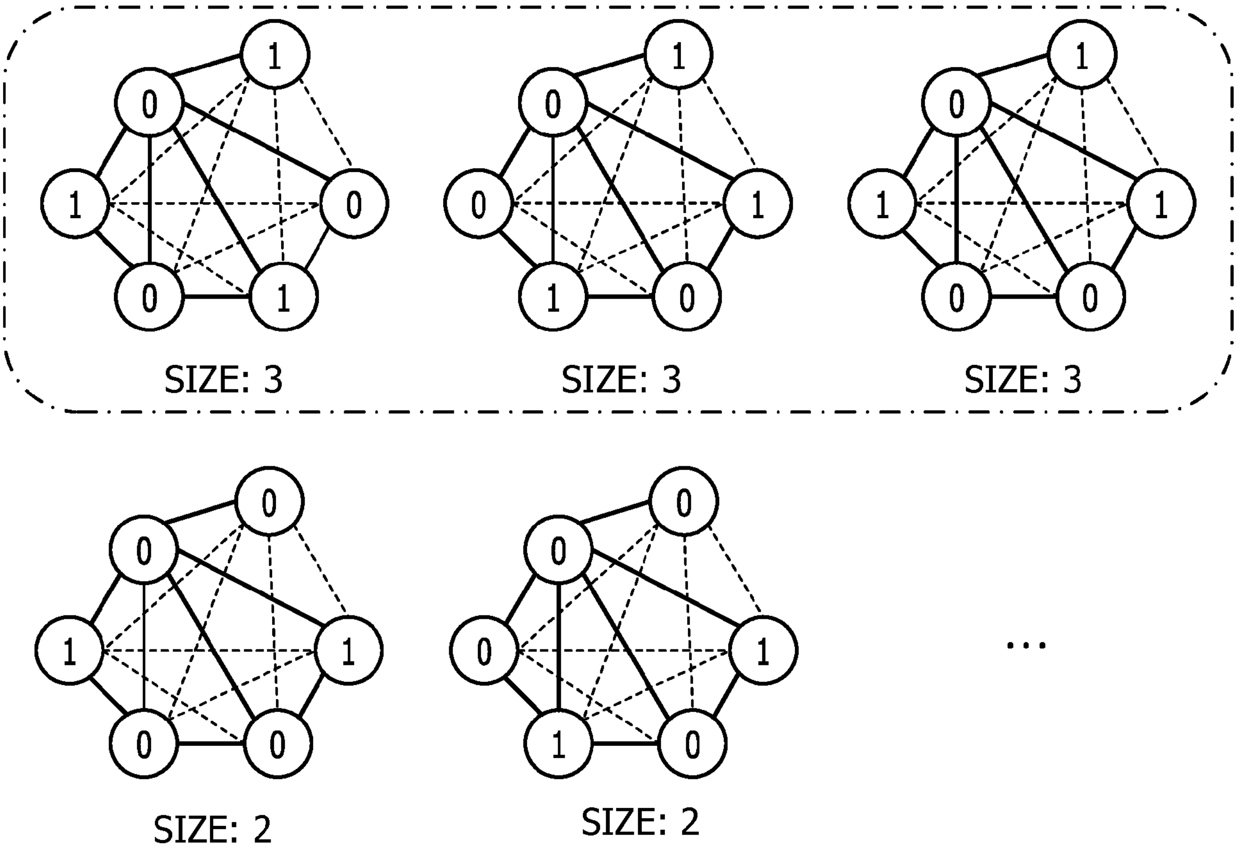

FIG. 6

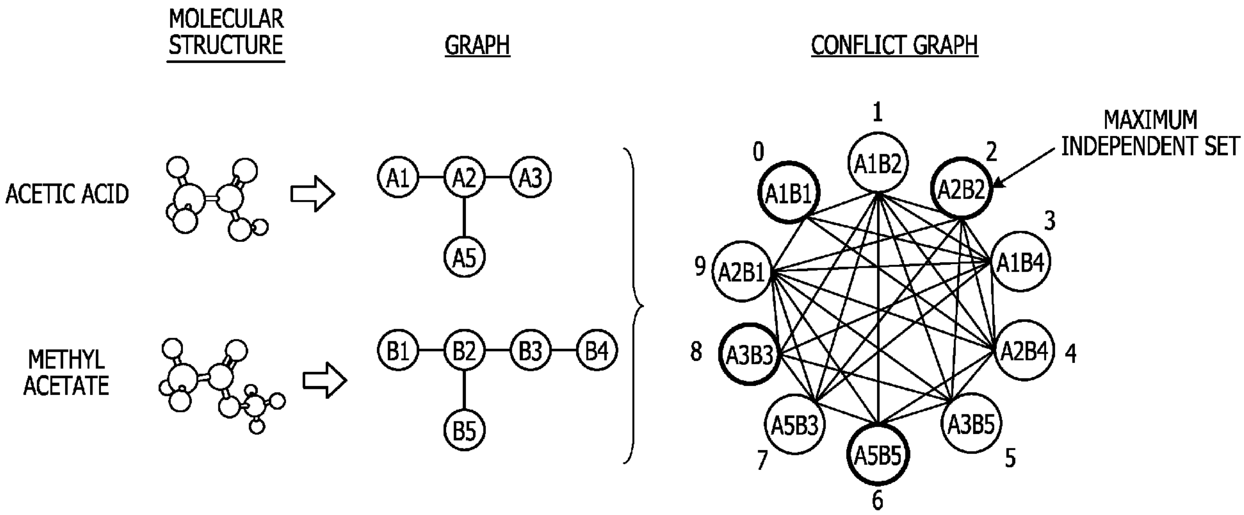
MOLECULAR STRUCTURE
GRAPH
CONFLICT GRAPH
ACETIC ACID
METHYL ACETATE
A1
A2
A3
A5
B1
B2
B3
B4
B5
MAXIMUM INDEPENDENT SET
0
1
2
3
4
5
6
7
8
9
A1B1
A1B2
A2B2
A1B4
A2B4
A3B5
A5B5
A5B3
A3B3
A2B1

FIG. 7

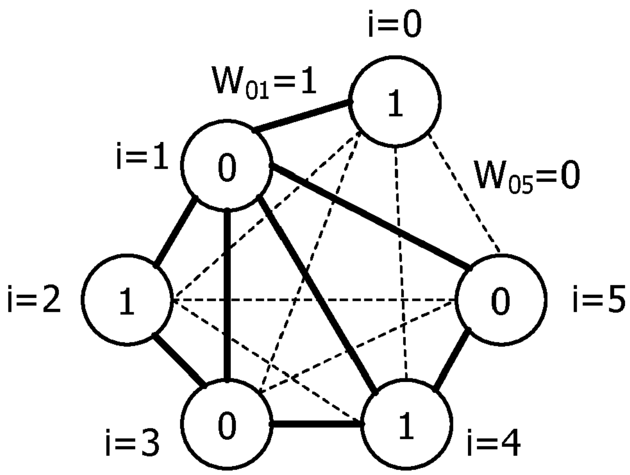


SIZE: 3

FIG. 8

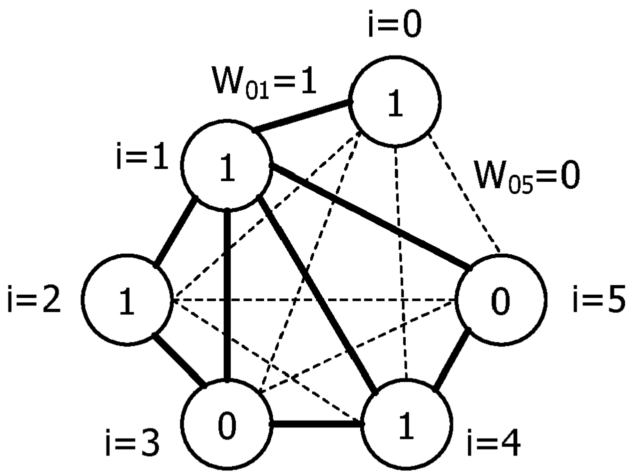


SIZE: 3

FIG. 9

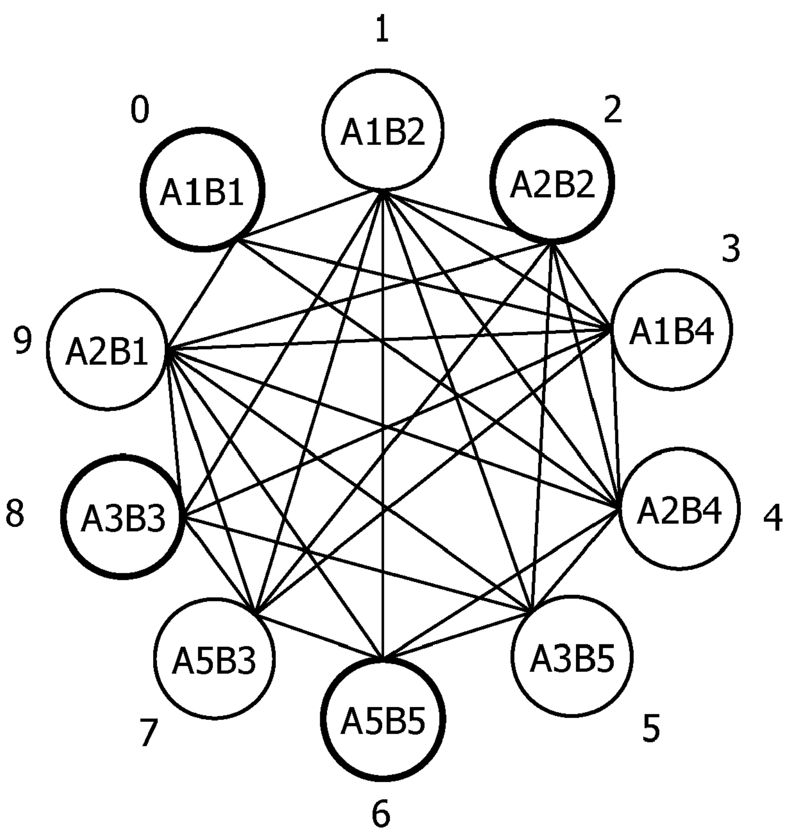
1
0
2
A1B2
A1B1
A2B2
3
9
A2B1
A1B4
8
A3B3
A2B4
4
A5B3
A3B5
7
A5B5
5
6

# FIG. 10

MOLECULE A:
ACETIC ACID

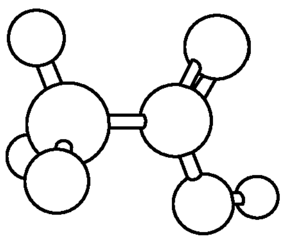

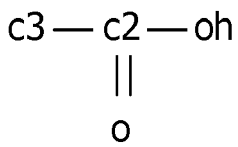

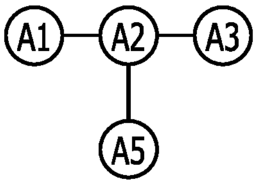


MOLECULE B:
METHYL ACETATE

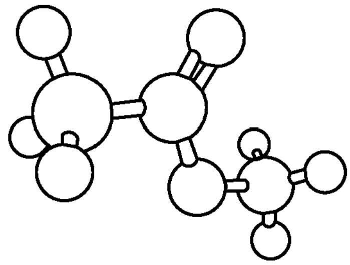

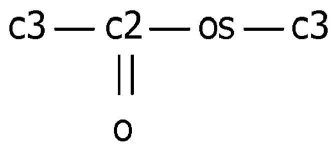

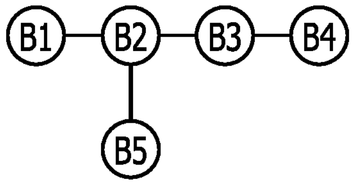

FIG. 11

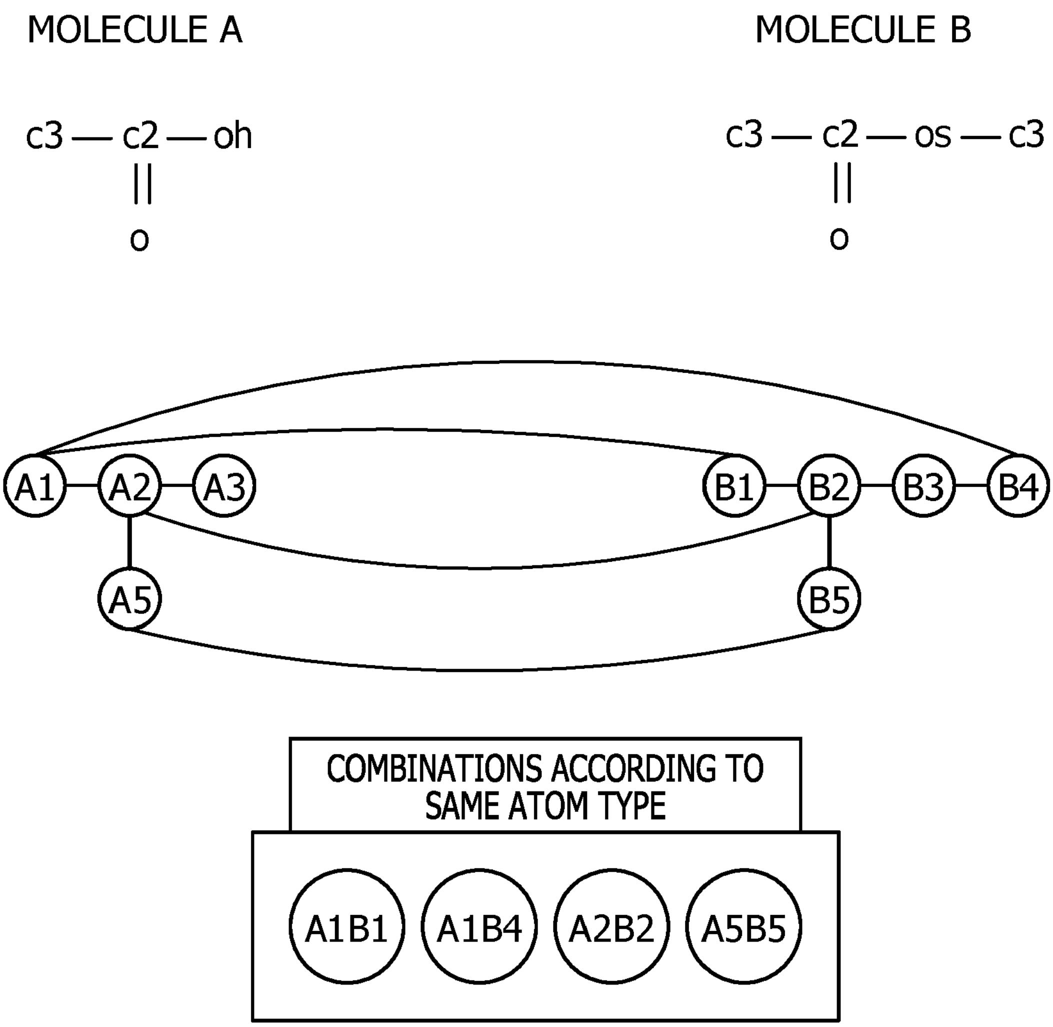
MOLECULE A
MOLECULE B
A1
A2
A3
A5
B1
B2
B3
B4
B5
COMBINATIONS ACCORDING TO SAME ATOM TYPE
A1B1
A1B4
A2B2
A5B5

FIG. 12

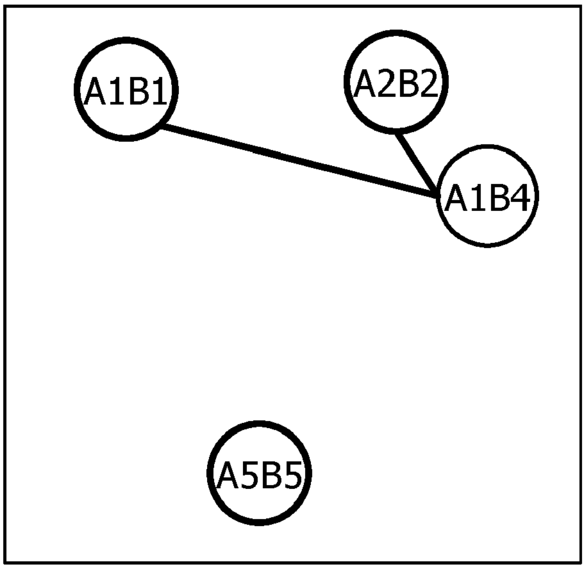

FIG. 13

(A) ACETIC ACID

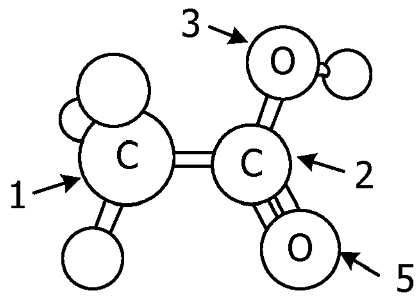

(B) METHYL ACETATE

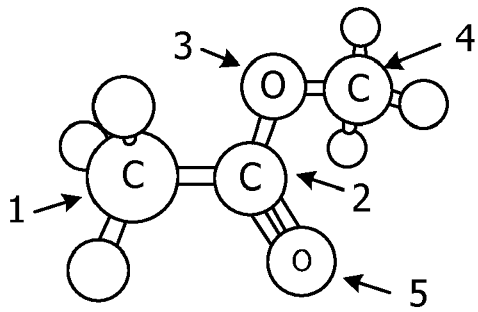

# FIG. 14

(A) ACETIC ACID

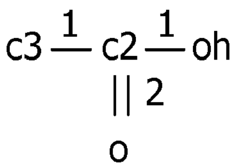

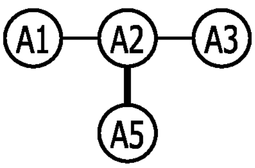


(B) METHYL ACETATE

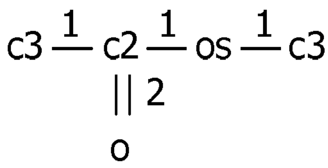

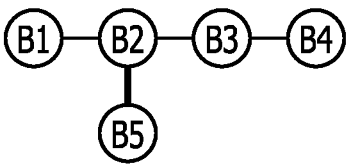

FIG. 15

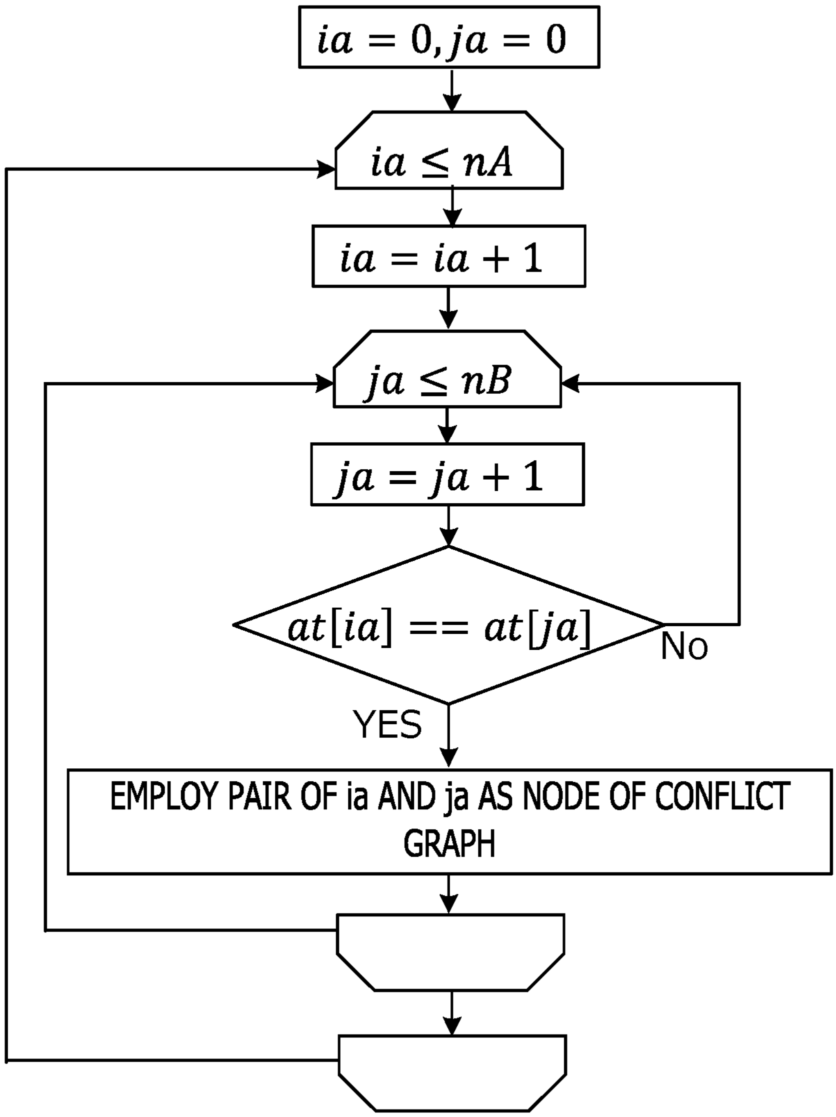
$ia = 0, ja = 0$
$ia \leq nA$
$ia = ia + 1$
$ja \leq nB$
$ja = ja + 1$
$at[ia] == at[ja]$
No
YES
EMPLOY PAIR OF ia AND ja AS NODE OF CONFLICT GRAPH

FIG. 16

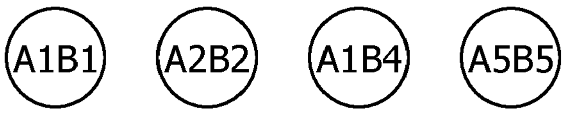

FIG. 17

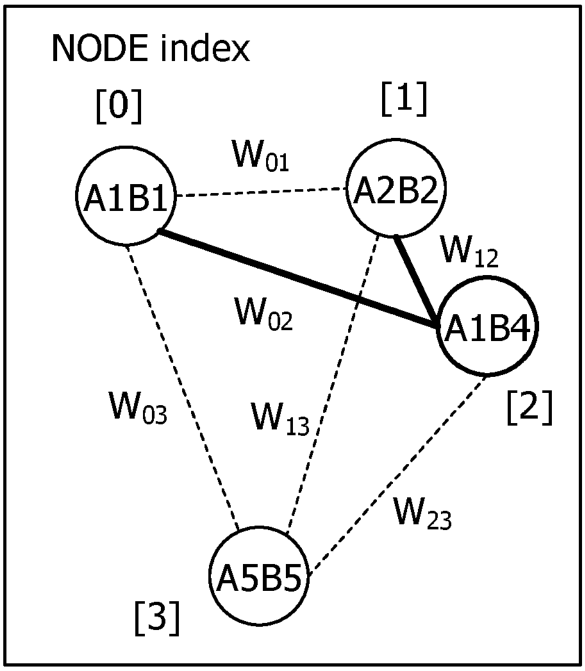

FIG. 18

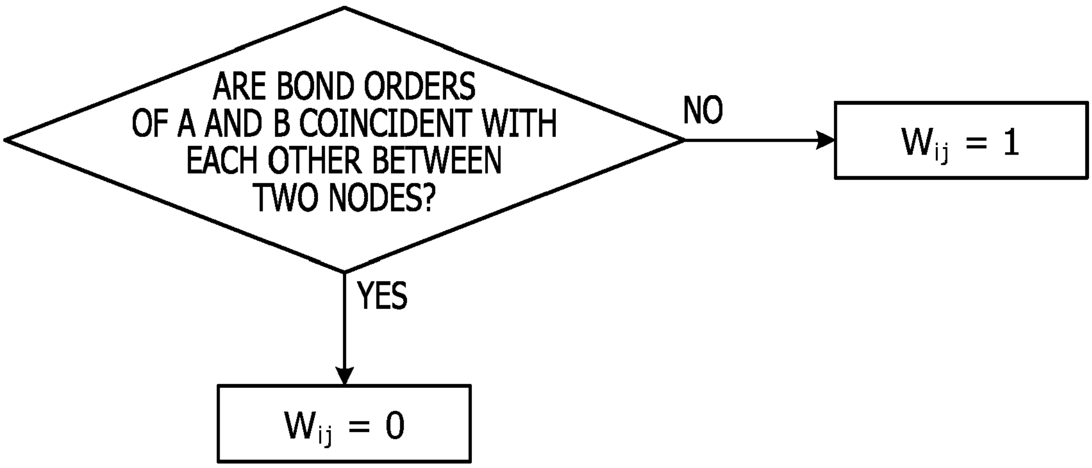
ARE BOND ORDERS OF A AND B COINCIDENT WITH EACH OTHER BETWEEN TWO NODES?
NO
$W_{ij} = 1$
YES
$W_{ij} = 0$

FIG. 19

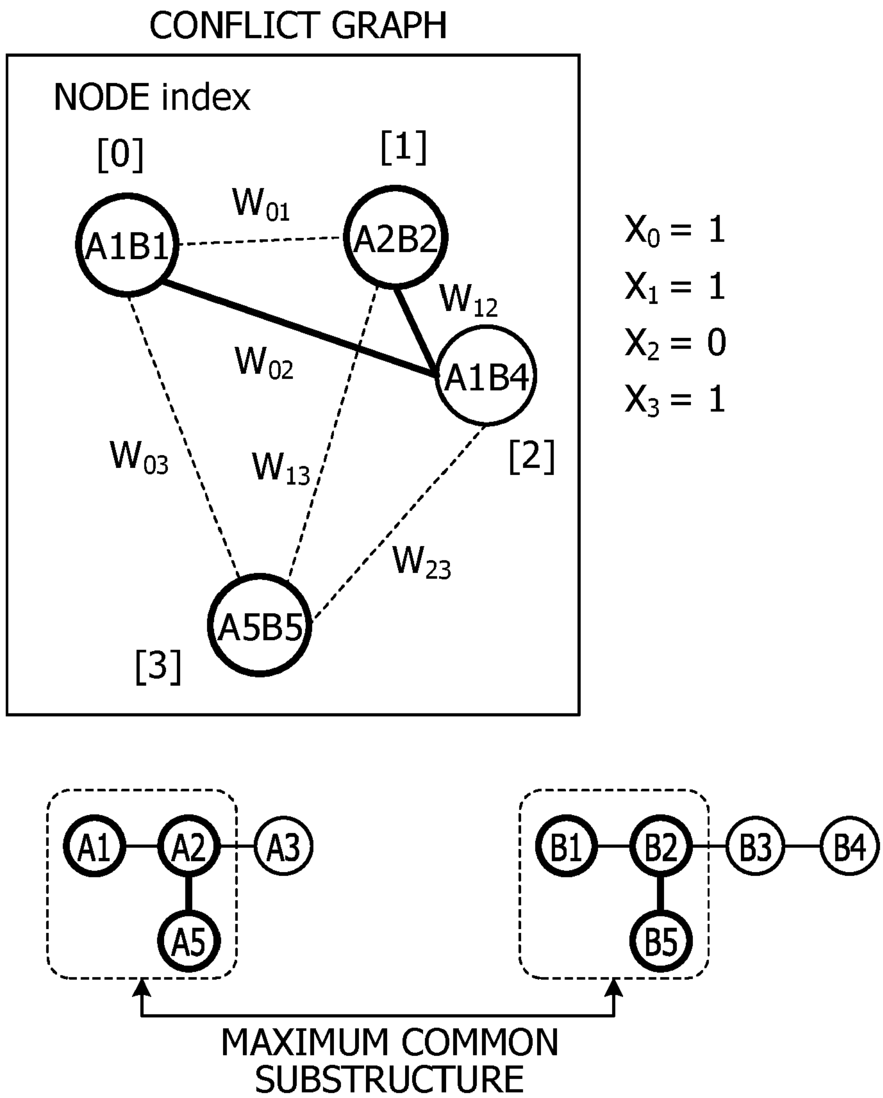
CONFLICT GRAPH
NODE index
[0]
[1]
[2]
[3]
A1B1
A2B2
A1B4
A5B5
$W_{01}$
$W_{02}$
$W_{03}$
$W_{12}$
$W_{13}$
$W_{23}$
$X_0 = 1$
$X_1 = 1$
$X_2 = 0$
$X_3 = 1$
A1
A2
A3
A5
B1
B2
B3
B4
B5
MAXIMUM COMMON
SUBSTRUCTURE

FIG. 20

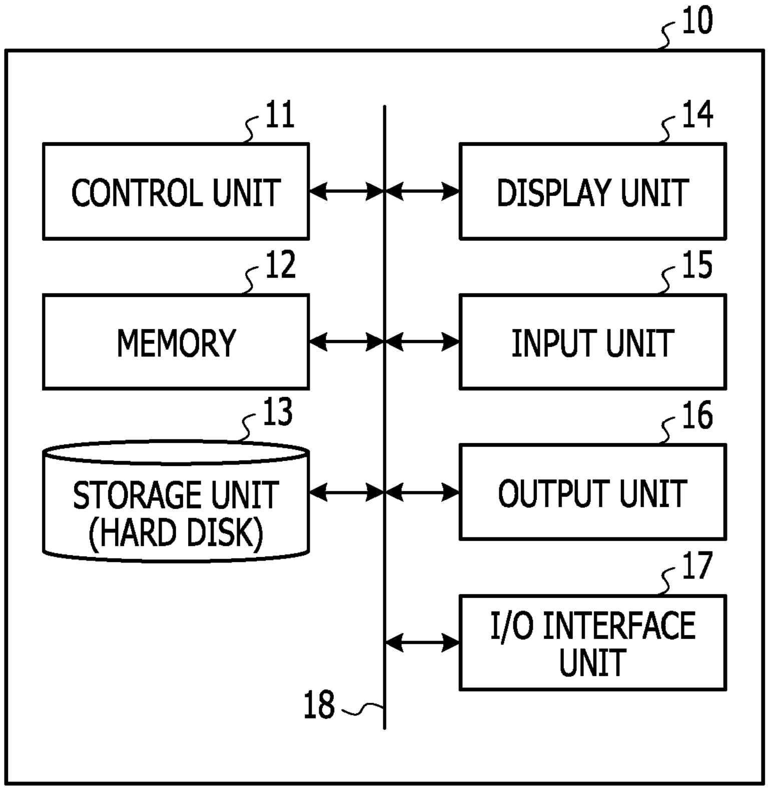
10
11
CONTROL UNIT
12
MEMORY
13
STORAGE UNIT
(HARD DISK)
14
DISPLAY UNIT
15
INPUT UNIT
16
OUTPUT UNIT
17
I/O INTERFACE
UNIT
18

FIG. 21

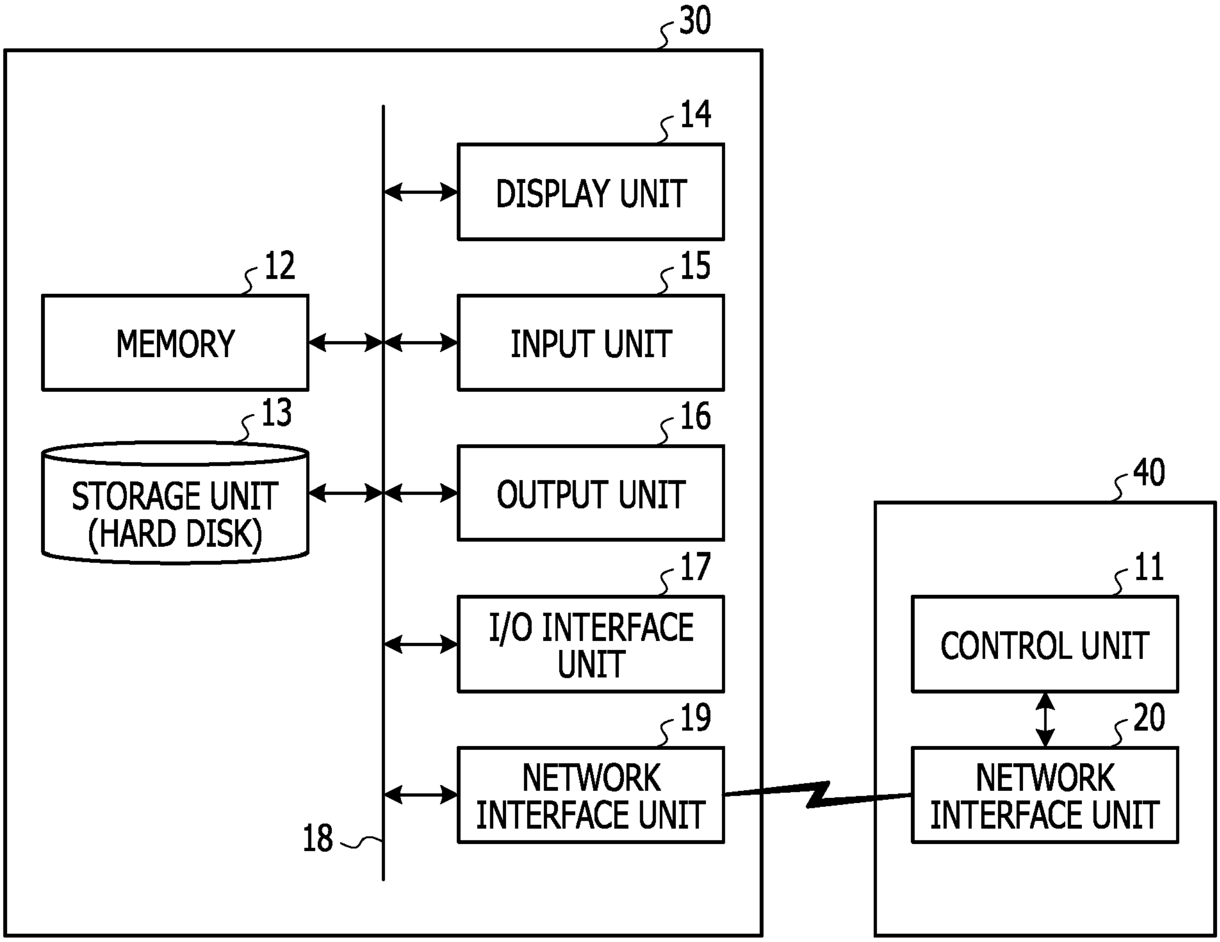
30
14
DISPLAY UNIT
12
MEMORY
15
INPUT UNIT
13
STORAGE UNIT (HARD DISK)
16
OUTPUT UNIT
17
I/O INTERFACE UNIT
19
NETWORK INTERFACE UNIT
18
40
11
CONTROL UNIT
20
NETWORK INTERFACE UNIT

FIG. 22

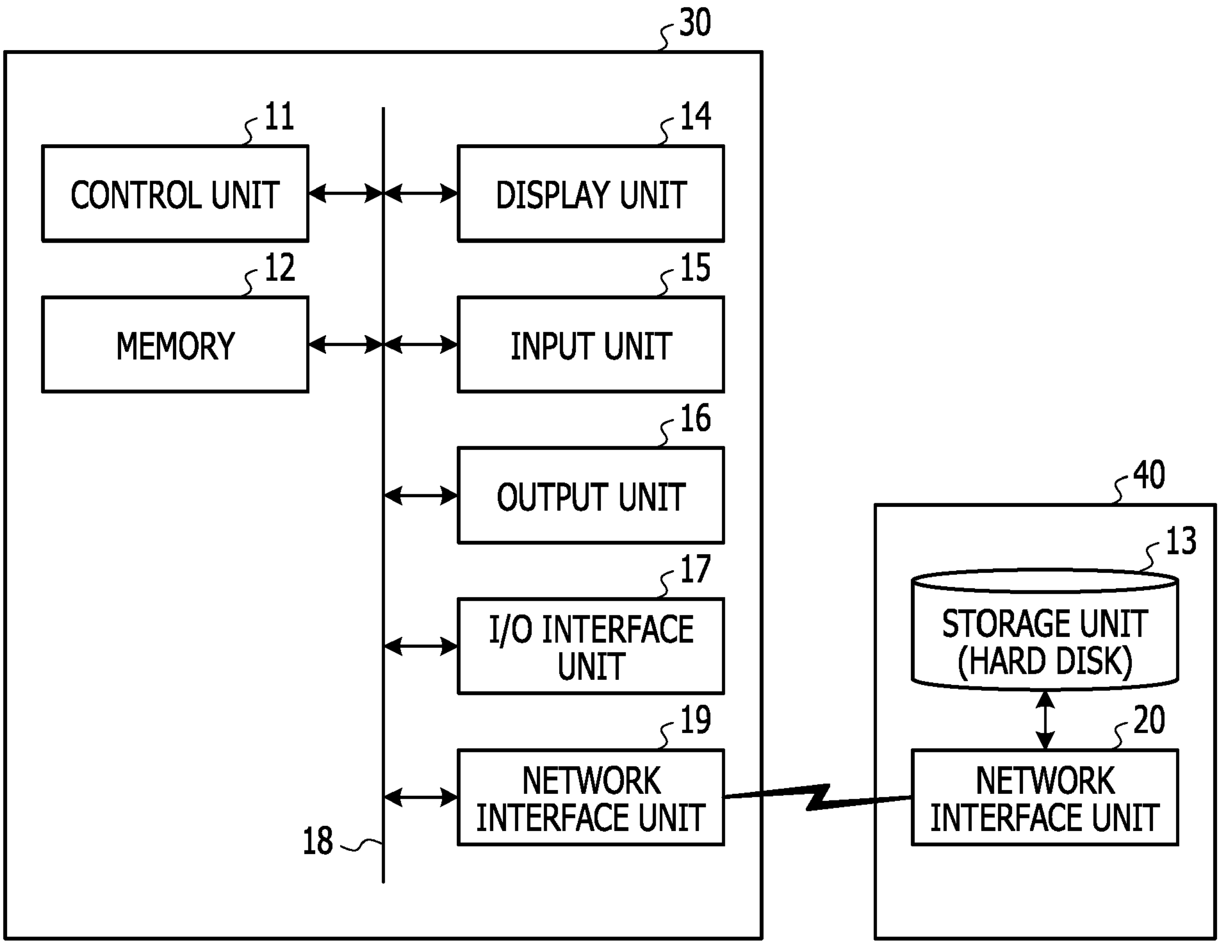
30
11
CONTROL UNIT
14
DISPLAY UNIT
12
MEMORY
15
INPUT UNIT
16
OUTPUT UNIT
40
13
STORAGE UNIT (HARD DISK)
17
I/O INTERFACE UNIT
19
20
NETWORK INTERFACE UNIT
NETWORK INTERFACE UNIT
18

FIG. 23

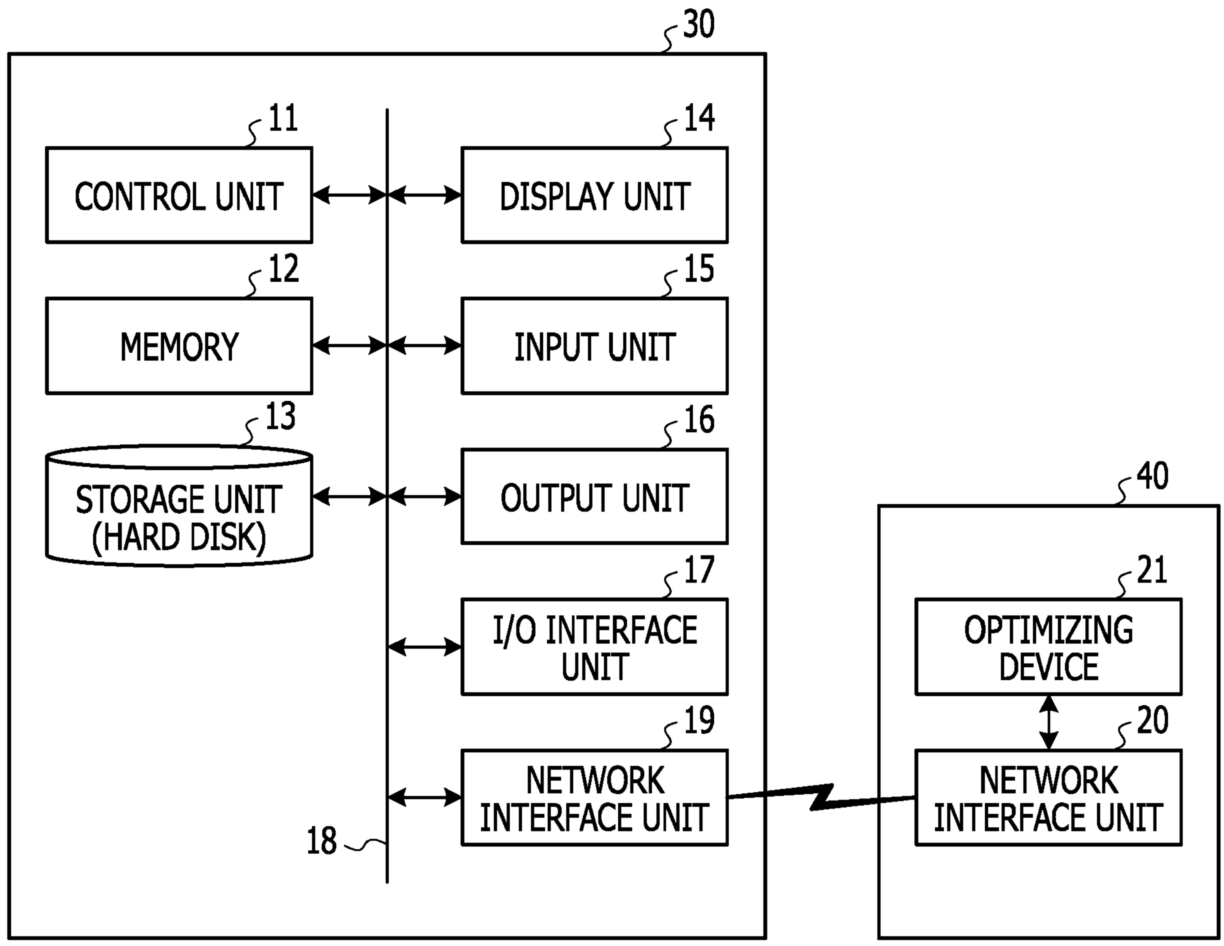
30
11
CONTROL UNIT
14
DISPLAY UNIT
12
MEMORY
15
INPUT UNIT
13
STORAGE UNIT (HARD DISK)
16
OUTPUT UNIT
17
I/O INTERFACE UNIT
19
NETWORK INTERFACE UNIT
18
40
21
OPTIMIZING DEVICE
20
NETWORK INTERFACE UNIT

FIG. 24

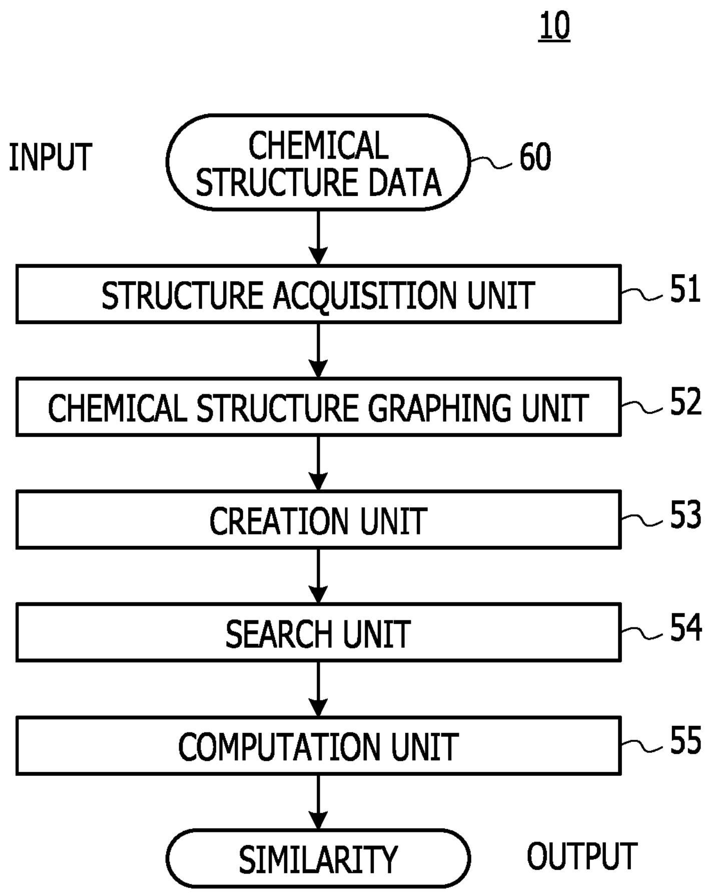
10
INPUT
CHEMICAL STRUCTURE DATA
60
STRUCTURE ACQUISITION UNIT
51
CHEMICAL STRUCTURE GRAPHING UNIT
52
CREATION UNIT
53
SEARCH UNIT
54
COMPUTATION UNIT
55
SIMILARITY
OUTPUT

FIG. 25

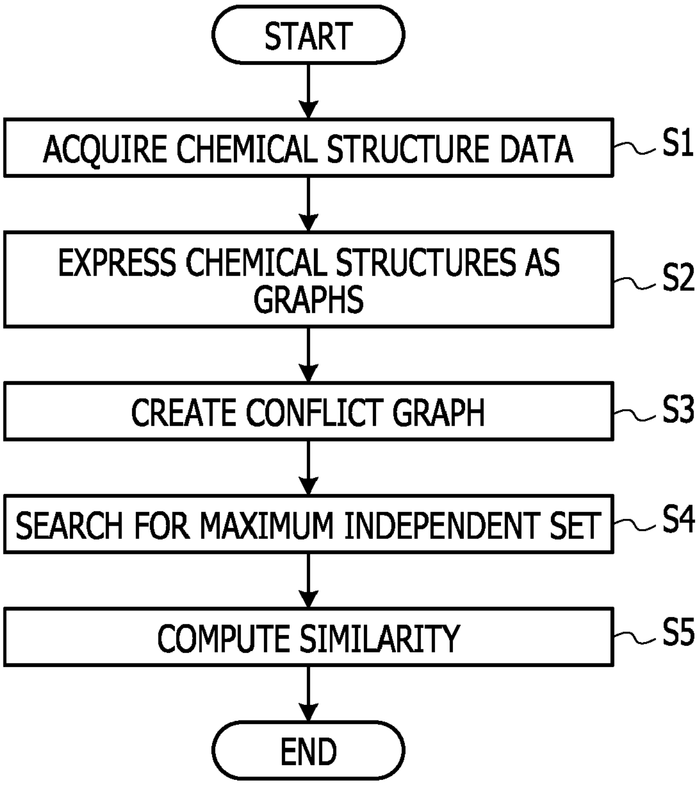
START
ACQUIRE CHEMICAL STRUCTURE DATA
S1
EXPRESS CHEMICAL STRUCTURES AS GRAPHS
S2
CREATE CONFLICT GRAPH
S3
SEARCH FOR MAXIMUM INDEPENDENT SET
S4
COMPUTE SIMILARITY
S5
END

FIG. 26

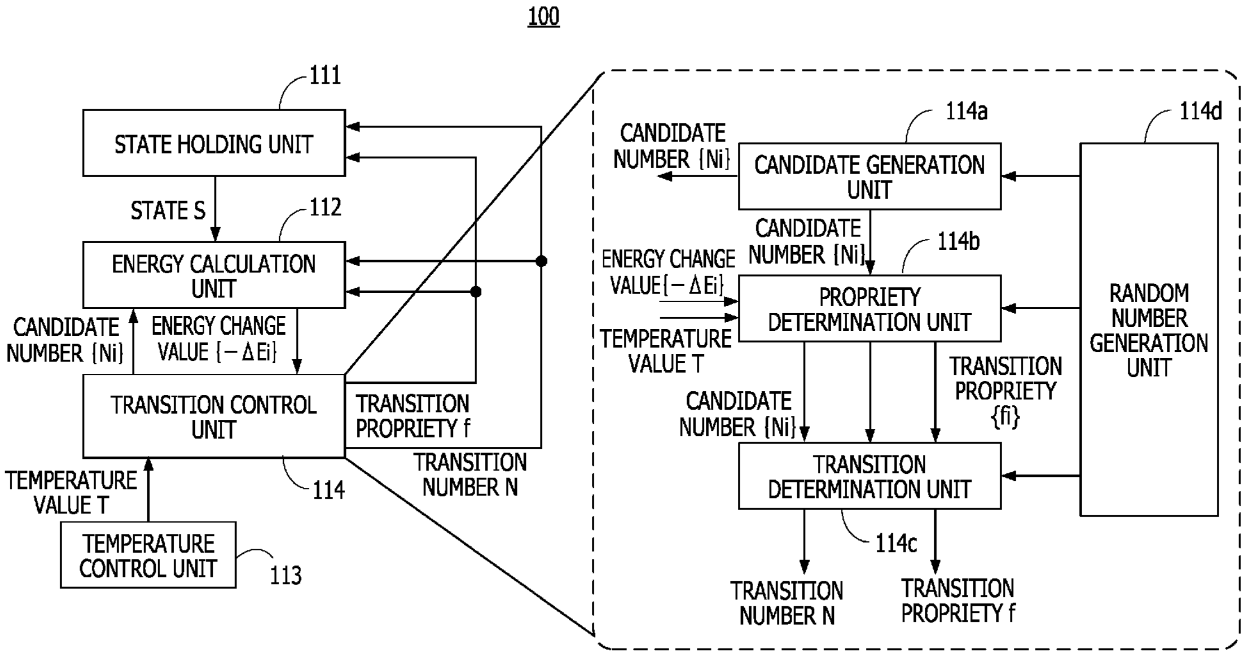
100
111
STATE HOLDING UNIT
STATE S
112
ENERGY CALCULATION UNIT
CANDIDATE NUMBER {Ni}
ENERGY CHANGE VALUE {−ΔEi}
TRANSITION CONTROL UNIT
TRANSITION PROPRIETY f
TRANSITION NUMBER N
114
TEMPERATURE VALUE T
TEMPERATURE CONTROL UNIT
113
114a
CANDIDATE NUMBER {Ni}
CANDIDATE GENERATION UNIT
114d
CANDIDATE NUMBER {Ni}
114b
ENERGY CHANGE VALUE{−ΔEi}
PROPRIETY DETERMINATION UNIT
TEMPERATURE VALUE T
RANDOM NUMBER GENERATION UNIT
TRANSITION PROPRIETY {fi}
CANDIDATE NUMBER {Ni}
TRANSITION DETERMINATION UNIT
114c
TRANSITION NUMBER N
TRANSITION PROPRIETY f

FIG. 27

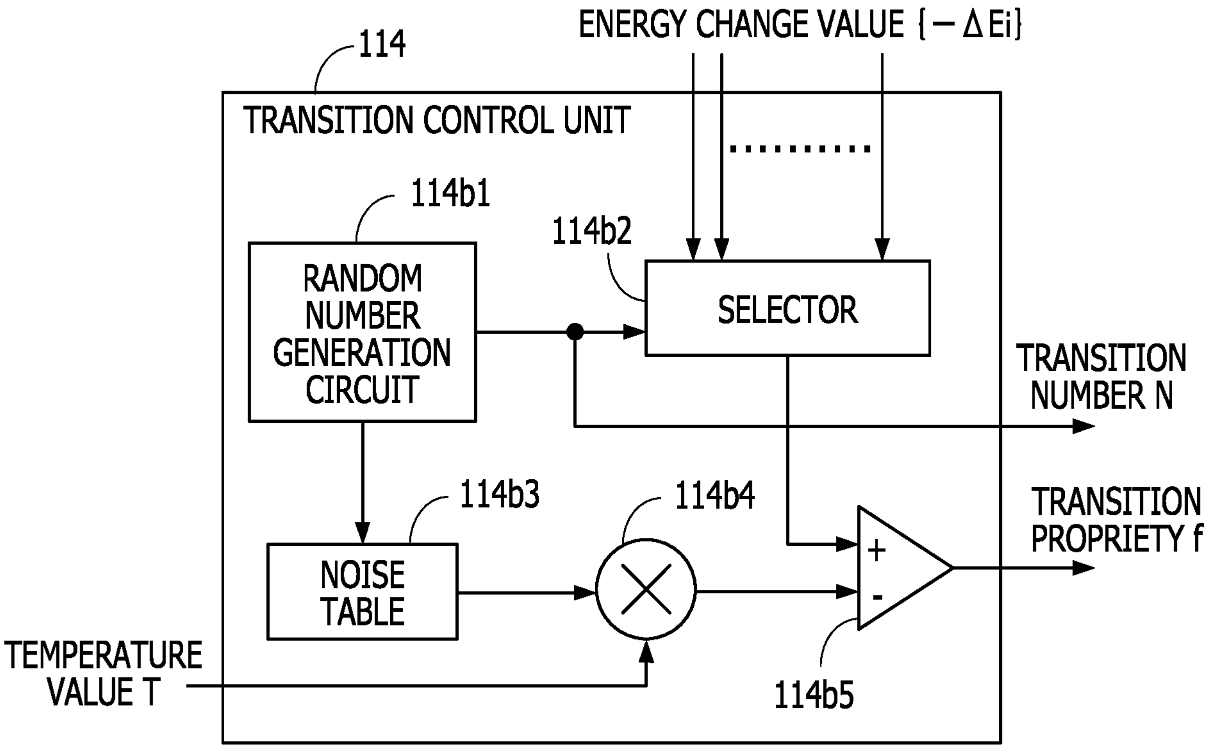
ENERGY CHANGE VALUE {−ΔEi}
114
TRANSITION CONTROL UNIT
114b1
114b2
RANDOM NUMBER GENERATION CIRCUIT
SELECTOR
TRANSITION NUMBER N
114b3
114b4
TRANSITION PROPRIETY f
NOISE TABLE
+
-
TEMPERATURE VALUE T
114b5

FIG. 28

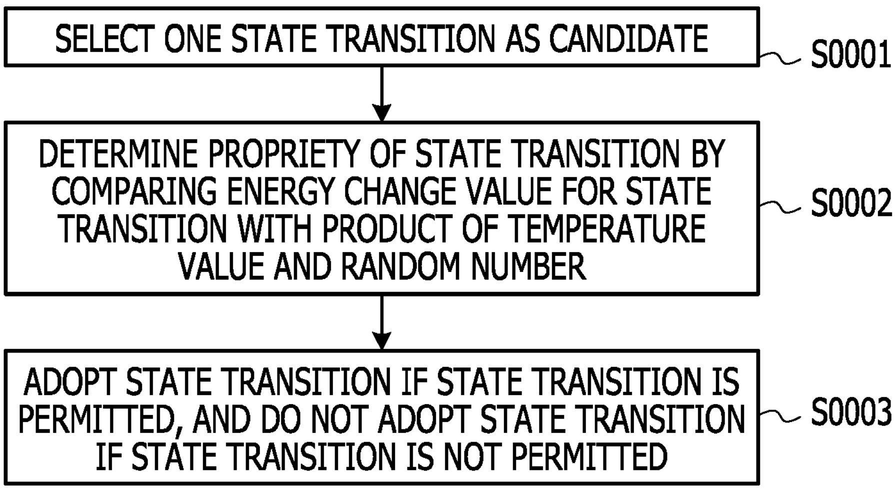
SELECT ONE STATE TRANSITION AS CANDIDATE
S0001
DETERMINE PROPRIETY OF STATE TRANSITION BY COMPARING ENERGY CHANGE VALUE FOR STATE TRANSITION WITH PRODUCT OF TEMPERATURE VALUE AND RANDOM NUMBER
S0002
ADOPT STATE TRANSITION IF STATE TRANSITION IS PERMITTED, AND DO NOT ADOPT STATE TRANSITION IF STATE TRANSITION IS NOT PERMITTED
S0003

FIG. 29

LINALOOL

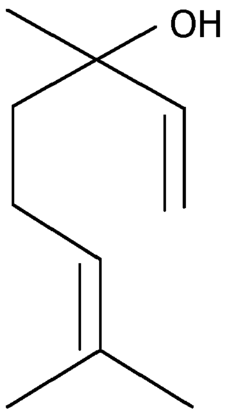

# FIG. 30

LINALOOL

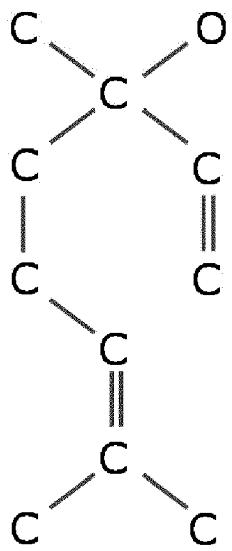

C: 10
O: 1

TERPINEOL

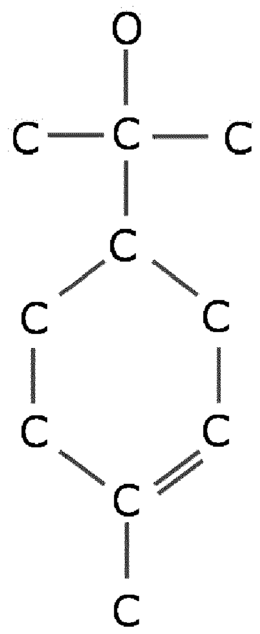

C: 10
O: 1

10*10+1*1 = 101 BITS

# FIG. 31

LINALOOL

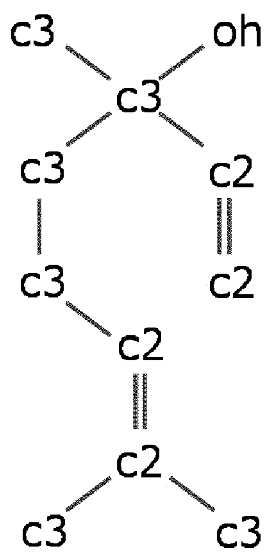

c3: 6
c2: 4
oh: 1

TERPINEOL

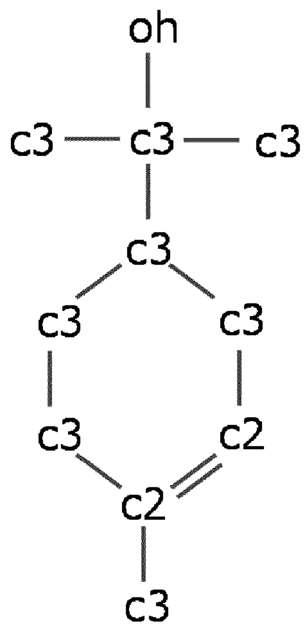

c3: 8
c2: 2
oh: 1

6*8+4*2+1*1 = 57 BITS

Europäisches Patentamt
European Patent Office
Office européen des brevets

# EUROPEAN SEARCH REPORT

Application Number
EP 20 20 5387

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HERNANDEZ MARITZA ET AL: "A Quantum-Inspired Method for Three-Dimensional Ligand-Based Virtual Screening", JOURNAL OF CHEMICAL INFORMATION AND MODELING, vol. 59, no. 10, 18 October 2019 (2019-10-18), pages 4475-4485, XP055793578, US ISSN: 1549-9596, DOI: 10.1021/acs.jcim.9b00195 Retrieved from the Internet: URL:https://pubs.acs.org/doi/pdf/10.1021/acs.jcim.9b00195> * abstract; figures 1,2,3; table 1 * * page 4477 - page 4478 * ----- | 1-13 | INV. G16C20/40 |
| X,D | MARITZA HERNANDEZ ET AL: "A Novel Graph-based Approach for Determining Molecular Similarity", ARXIV.ORG, CORNELL UNIVERSITY LIBRARY, 201 OLIN LIBRARY CORNELL UNIVERSITY ITHACA, NY 14853, 25 January 2016 (2016-01-25), XP080680147, * abstract * * pages 3-7 * ----- | 1-13 | TECHNICAL FIELDS SEARCHED (IPC): G16C G06F |
| A | US 2011/225148 A1 (PITMAN MICHAEL C [US] ET AL) 15 September 2011 (2011-09-15) * claims 1-12 * ----- | 1-13 | |

The present search report has been drawn up for all claims

1

| Place of search | Date of completion of the search | Examiner |
|---|---|---|
| The Hague | 8 April 2021 | Nurmi, Jussi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 20 20 5387

This annex lists the patent family members relating to the patent documents cited in the above-mentioned European search report. The members are as contained in the European Patent Office EDP file on
The European Patent Office is in no way liable for these particulars which are merely given for the purpose of information.

08-04-2021

| Patent document cited in search report | Publication date | Patent family member(s) | Publication date |
|---|---|---|---|
| US 2011225148 A1 | 15-09-2011 | US 2003009298 A1 | 09-01-2003 |
| | | US 2010057797 A1 | 04-03-2010 |
| | | US 2011225148 A1 | 15-09-2011 |
| | | US 2013036120 A1 | 07-02-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HERNANDEZ, MARITZA ; ZARIBAFIYAN, ARMAN ; ARAMON, MALIHEH ; NAGHIBI, MOHAMMAD.** *A Novel Graph-based Approach for Determining Molecular Similarity, https://arxiv.org/pdf/1601.06693.pdf*
- **WANG, JUNMEI ; WOLF, ROMAIN M. ; CALDWELL, JAMES W. ; KOLLMAN, PETER A. ; CASE, DAVID A.** Development and Testing of a General Amber Force Field. *Journal of Computational Chemistry,* vol. 25 (9
- **JAKALIAN, ARAZ ; JACK, DAVID B. ; BAYLY, CHRISTOPHER I.** Fast, Efficient Generation of High-Quality Atomic Charges. AM1-BCC Model: II. Parameterization and Validation. *Journal of Computational Chemistry,* 2002, vol. 23, 1623-1641